(19) Europäisches Patentamt / European Patent Office / Office européen des brevets

(11) **EP 1 977 676 A1**

(12) **EUROPEAN PATENT APPLICATION**
published in accordance with Art. 158(3) EPC

(43) Date of publication:
**08.10.2008 Bulletin 2008/41**

(51) Int Cl.:
*A61B 1/00* (2006.01)      *G06T 1/00* (2006.01)
*G06T 7/20* (2006.01)      *A61B 5/07* (2006.01)

(21) Application number: **06832704.8**

(22) Date of filing: **16.11.2006**

(86) International application number:
**PCT/JP2006/322815**

(87) International publication number:
**WO 2007/083436 (26.07.2007 Gazette 2007/30)**

(84) Designated Contracting States:
**DE FR GB**

(30) Priority:   **23.01.2006   JP 2006014444**

(71) Applicant: **Olympus Medical Systems Corp.**
**Tokyo 151-0072 (JP)**

(72) Inventors:
• **HASEGAWA, Jun**
**c/o OLYMPUS MEDICAL SYSTEMS CORP.**
**Tokyo 151-0072 (JP)**

• **NONAMI, Tetsuo**
**c/o OLYMPUS MEDICAL SYSTEMS CORP.**
**Tokyo 151-0072 (JP)**

(74) Representative: **von Hellfeld, Axel et al**
**Wuesthoff & Wuesthoff**
**Patent- und Rechtsanwälte**
**Schweigerstrasse 2**
**81541 München (DE)**

(54) **CAPSULE-TYPE MEDICAL DEVICE, CONTROL DEVICE FOR MEDICAL USE, IMAGE PROCESSING DEVICE FOR MEDICAL USE, AND PROGRAM**

(57)     A capsule medical device of the invention comprises: an image pickup unit that captures an image of a subject, and outputs the image of the subject as an image pickup signal; and a control unit that determines with respect to similarity among a plurality of images contained in an image group in accordance with the respective image pickup signals of two or more frames among those output from the image pickup unit based on a predetermined threshold value indicating a magnitude of fluctuation between the images contained in the image group, and controls to output an image signal based on a result of the determination.

**FIG.5**

EP 1 977 676 A1

## Description

Technical Field

The present invention relates to a capsule medical device, a medical control device, a medical image processing device and a program, and more particularly to a capsule medical device, a medical control device, a medical image processing device and a program which allow image pickup signals or image signals to be output in accordance with similarity between images based on the image pickup signals or the image signals of two or more frames.

Background Art

A medical system which processes an image signal or a video signal output from a medical device including an image pickup unit disposed within a live body for picking up the inside image of the live body, and displays the image of the live body based on the processed image or video signals has been widely employed for observing the inside the body.

The medical capsule system disclosed in Japanese Unexamined Patent Application Publication No. 2004-000645 has been proposed as the aforementioned medical system for example. The medical capsule system disclosed in Japanese Unexamined Patent Application Publication No. 2004-000645 is structured to allow the personal computer to process video signals output from the medical capsule device (hereinafter referred to as a capsule medical device) disposed within the live body and equipped with an observation unit for picking up the inside image of the live body, and to display the inside image of the live body on the monitor based on the processed video signals as the moving image or the static image in the form of the frame of the moving image. The aforementioned medical capsule system is structured to allow the image storage unit to store the inside image of the live body displayed on the monitor. With the use of the medical capsule system structured as described above, the operator is allowed to diagnose the live body while observing the moving image or the static image in the form of the frame of the moving image stored in the image storage unit.

The medical capsule device advances by peristaltic motion of a digestive tract, so that it normally takes about several hours after the device is put in a live body from a mouse until the device is discharged from an anus. In addition, since the medical capsule device almost constantly outputs image signals or video signals after being put in the live body until being discharged from the live body, the number of still images as frame images in a moving image accumulated for several hours is enormous. When an operator finds out a part picking up an image of an abnormal region in the live body from the moving image for several hours, the operator has to make a diagnosis while viewing all the stored frame images one by one, including the frame images containing several picked-up images of a normal region or the same abnormal region. As a result, the operator is forced to shoulder a burden when making a diagnosis of a live body. However, there is no proposal to the above-described problem in the medical capsule system proposed in the Japanese Unexamined Patent Application Publication No. 2004-000645.

The present invention has been made in view of the above-described points, and an object of the present invention is to provide a capsule medical device, a medical control device, a medical image processing device and a program which are capable of reducing a burden when an operator or the like observes a live body by allowing image signals to be output based on similarity between respective images of two or more frames.

Disclosure of Invention

Means for Solving the Problem

A first capsule medical device of the present invention is provided with an image pickup unit that captures an image of a subject and outputs the image of the subject as an image pickup signal, and a control unit that determines with respect to similarity between a plurality of images contained in an image group in accordance with the image pickup signals of two or more frames among those output from the image pickup unit based on a predetermined threshold value indicating a magnitude of fluctuation between the images contained in the image group, and controls to output an image signal based on a result of the determination.

A second capsule medical device of the present invention is the first capsule medical device, wherein the control unit determines with respect to the similarity between adjacent images of those contained in the image group based on the predetermined threshold value, and extracts the image pickup signal of one frame from the image pickup signals of the two or more consecutive frames based on the similarity so as to be output.

A third capsule medical device of the present invention is the first or the second capsule medical device, wherein the predetermined threshold value corresponds with an average value of a displacement value that indicates a displacement amount of a portion of the image at a previous time point in a portion of the other image at a subsequent time point among the images contained in the image group.

A fourth capsule medical device of the present invention is any one of the first to the third capsule medical device, wherein the image pickup unit includes a selector portion that selectively converts the image pickup signal into an image signal so as to be output.

A fifth capsule medical device of the present invention is any one of the first to the third capsule medical device, wherein the control unit determines with respect to the similarity between the images contained in the image group by making a determination of a threshold value based on a similarity value that indicates the similarity between a portion of the image at the previous time point and a portion of the other image at the subsequent time point in the images contained in the image group.

A sixth capsule medical device of the present invention is any one of the first to the third capsule medical device, wherein the control unit divides the image contained in the image group into a plurality of areas to calculate a motion vector and an angle formed by the motion vector in each of the divided areas, and determines with respect to the similarity between the images contained in the image group by making a determination of the threshold value based on a number of occurrences of the motion vector in a histogram of the angle.

A seventh capsule medical device of the present invention is any one of the first to the third capsule medical device, wherein the control unit determines with respect to the similarity between the images contained in the image group by making a determination of a threshold value based on an average value of magnitudes of the motion vectors of the respective images contained in the image group.

A first medical control device is provided with a selector unit for selectively outputting an image signal output from a medical device equipped with an image pickup unit that outputs a captured subject image as the image signal, and a control unit for determining with respect to a similarity between images among a plurality of images contained in an image group in accordance with the image signals of two or more frames among those output from the medical device based on a predetermined threshold value indicating a fluctuation between the images contained in the image group, and controls to allow the selector unit to output the image signal based on a result of the determination.

A second medical control device of the present invention is the first medical control device, wherein the control unit determines with respect to the similarity between adjacent images of the images contained in the image group based on the predetermined threshold value, and controls to extract the image signal of one frame from the image signals of the two or more consecutive frames based on the similarity so as to allow the selector unit to output the extracted image signal.

A third medical control device of the present invention is the first or the second medical control device, wherein the predetermined threshold value corresponds with an average value of a displacement value that indicates a displacement amount of a portion of the image at a previous time point in a portion of the other image at a subsequent time point among the images contained in the image group.

A fourth medical control device of the present invention is any one of the first to the third medical control device, further comprises a storage unit for storing the image signal output from the image pickup unit, wherein the control unit executes the predetermined control with respect to the image signal stored in the storage unit.

A first medical image processing device of the present invention is provided with a selector unit for selectively outputting an image signal output from a medical control device for controlling a medical device equipped with an image pickup unit that outputs a captured subject image as the image signal, and a control unit for determining with respect to similarity between images among a plurality of images contained in an image group in accordance with the image pickup signal of two or more frames among those output from the medical control device based on a predetermined threshold value indicating a fluctuation between the images contained in the image group, and executes a predetermined control to allow the selector unit to output the image signal based on a result of the determination.

A second medical image processing device of the present invention is the first medical image processing device, wherein the control unit determines with respect to the similarity between adjacent images of the images contained in the image group based on the predetermined threshold value, and controls to extract the image signal of one frame from the image signals of the two or more consecutive frames based on the result of the determination so as to allow the selector unit to output the extracted image signal.

A third medical image processing device of the present invention is the first medical image processing device, wherein the control unit determines with respect to the similarity between adjacent images of the images contained in the image group based on the predetermined threshold value; and when a level of the similarity between one image of the adjacent images and the other consecutive image at a subsequent time point is low, the control unit controls to extract the one image, and to insert a predetermined number of sheets of the one image between the one image and the subsequent image to be output from the selector unit.

A fourth medical image processing device of the present invention is any one of the first to the third medical image processing device, wherein the predetermined threshold value corresponds with an average value of a displacement value that indicates a displacement amount of a portion of the image at a previous time point in a portion of the other image at a subsequent time point among the images contained in the image group.

A fifth medical image processing device of the present invention is any one of the first to the fourth medical image processing device, further comprises a storage unit for storing the image signal output from the medical control device, wherein the control unit executes the predetermined control with respect to the image signal stored in the storage unit.

A first program of the present invention allows a computer that processes an image signal captured by a medical device equipped with the image pickup unit to execute a similarity detection procedure for determining with respect to the similarity between adjacent images of an image group including a plurality of images based on the image signals of two or more frames among those obtained by the medical device based on a predetermined threshold value that indicates the fluctuation between the images of the image group, and an image signal extraction procedure extracting and outputting the image signal of one frame from those of two or more consecutive frames.

A second program of the present invention is the first program, wherein the predetermined threshold value corresponds with an average value of a displacement value that indicates a displacement amount of a portion of the image at a previous time point in a portion of the other image at a subsequent time point among the images contained in the image group.

Brief Description of the Drawings

Fig. 1 is a view schematically showing a medical image processing system according to a first embodiment.

Fig. 2 is a view showing an exemplary state where an external unit according to the first embodiment is connected to a terminal.

Fig. 3 is a view showing an exemplary inner structure of a capsule medical device according to the first embodiment.

Fig. 4 is a block diagram of each structure of the external unit and the terminal according to the first embodiment.

Fig. 5 is a flowchart of an exemplary process, different from the one shown in Fig. 4, executed by the external unit according to the first embodiment with respect to image signals output from the capsule medical device.

Fig. 6 is a flowchart of another exemplary process, different from those shown in Figs. 4 and 5, executed by the external unit according to the first embodiment with respect to image signals output from the capsule medical device.

Fig. 7 is a flowchart of another exemplary process, different from those shown in Figs. 4, 5 and 6, executed by the external unit according to the first embodiment with respect to image signals output from the capsule medical device.

Fig. 8 is a flowchart of another exemplary process, different from those shown in Figs. 4, 5, 6 and 7, executed by the external unit according to the first embodiment with respect to image signals output from the capsule medical device.

Fig. 9 is a flowchart of another exemplary process, different from those shown in Figs. 4, 5, 6, 7 and 8, executed by the external unit according to the first embodiment with respect to image signals output from the capsule medical device.

Fig. 10 is a view showing an exemplary process for detecting the position where an area of the image on a previous frame becomes most adaptable to the image on the current frame.

Fig. 11 is a view graphically showing an angle based on a direction of a motion vector.

Fig. 12 is a flowchart of an exemplary process executed by a terminal according to a second embodiment with respect to image signals stored in a storage circuit.

Fig. 13 is a flowchart of an exemplary process executed by a terminal according to a third embodiment with respect to image signals stored in a storage circuit.

Fig. 14 is a view showing an exemplary histogram of angles of the motion vectors.

Fig. 15 is a view showing exemplary images displayed on the monitor where the process shown in Fig. 12 has not been executed.

Fig. 16 is a view graphically showing the process of a group of images with small fluctuation selected among captured images as one image group in the process shown in Fig. 12.

Fig. 17 is a view showing exemplary images displayed on the monitor in the case where the process shown in Fig. 12 has been executed.

Fig. 18 is a view showing a state where motion vectors are connected.

Fig. 19 is a view showing exemplary images displayed on the monitor in the case where the process shown in Fig. 13 has not been executed.

Fig. 20 is a view graphically showing the process of a group of images with small fluctuation selected among captured images as one image group in the process shown in Fig. 13.

Fig. 21 is a view showing exemplary images displayed on the monitor where the process shown in Fig. 13 has been executed.

**EP 1 977 676 A1**

Best Mode for Carrying Out the Invention

Below, embodiments of the present invention will be described referring to the drawings.

(First Embodiment)

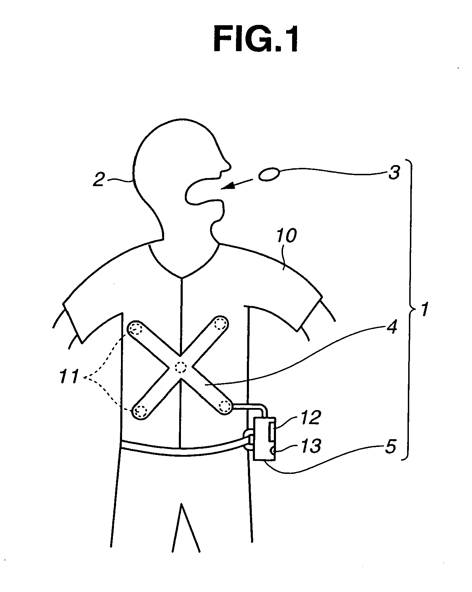

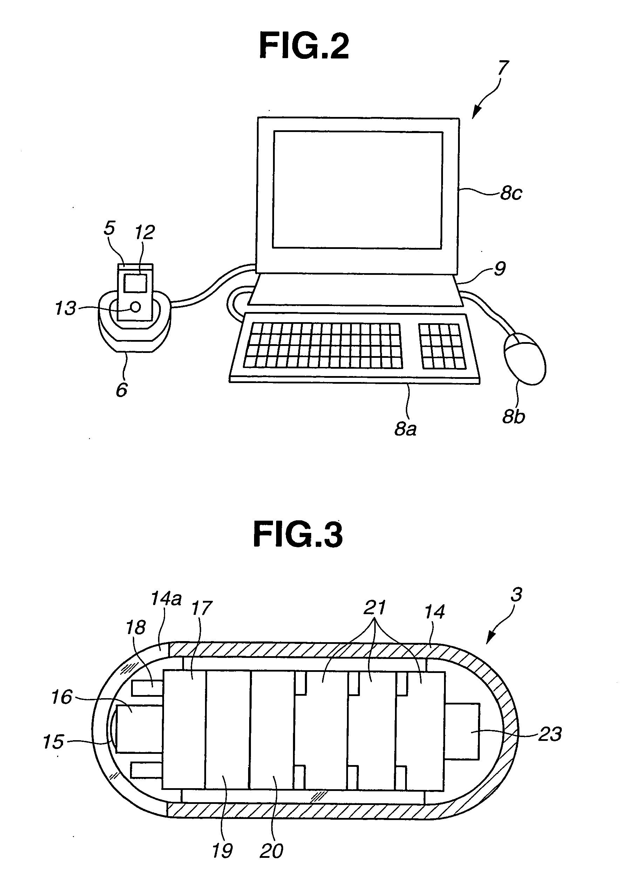

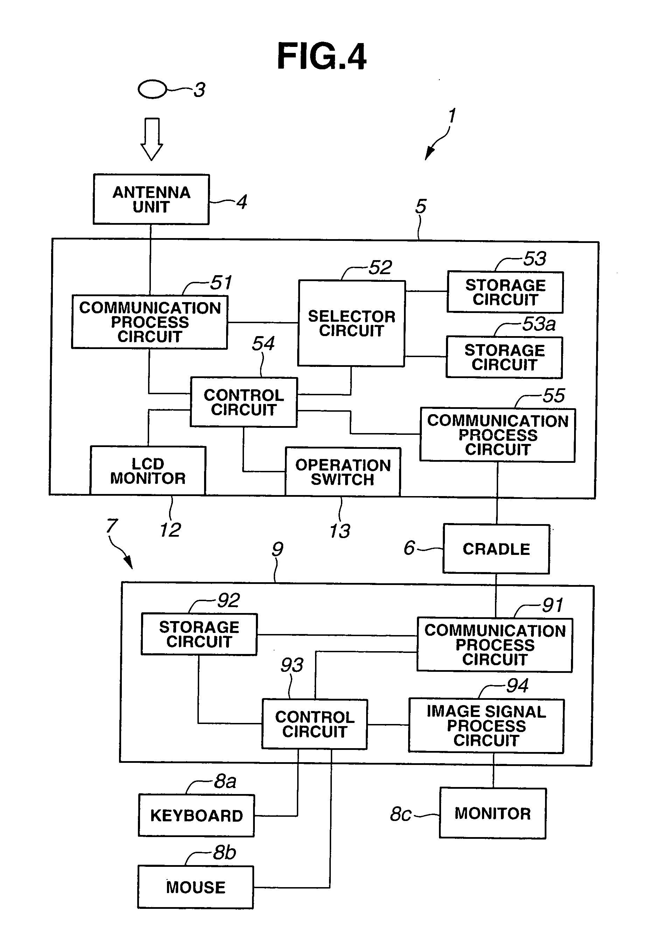

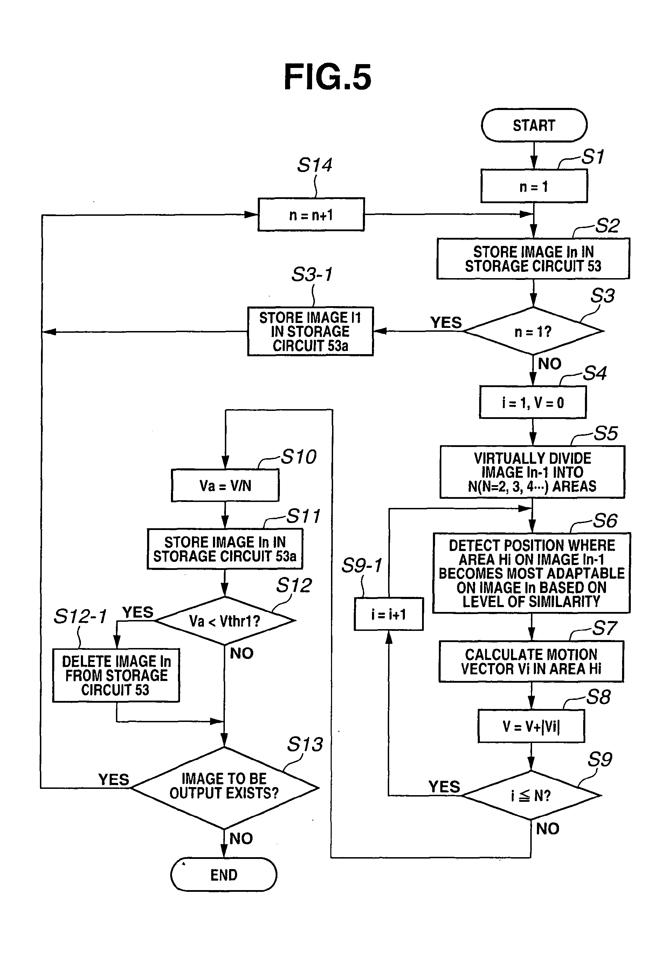

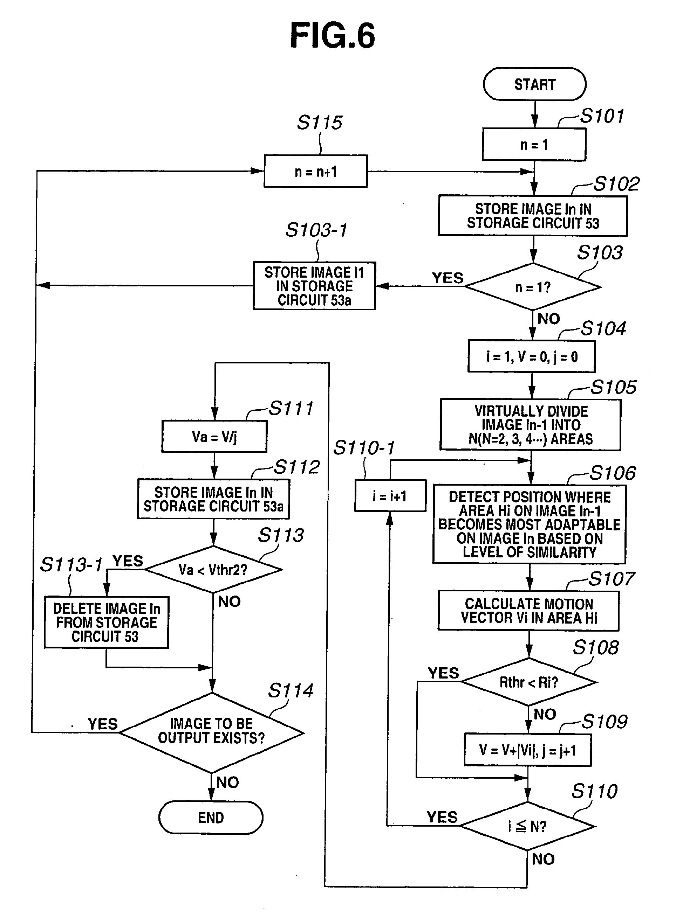

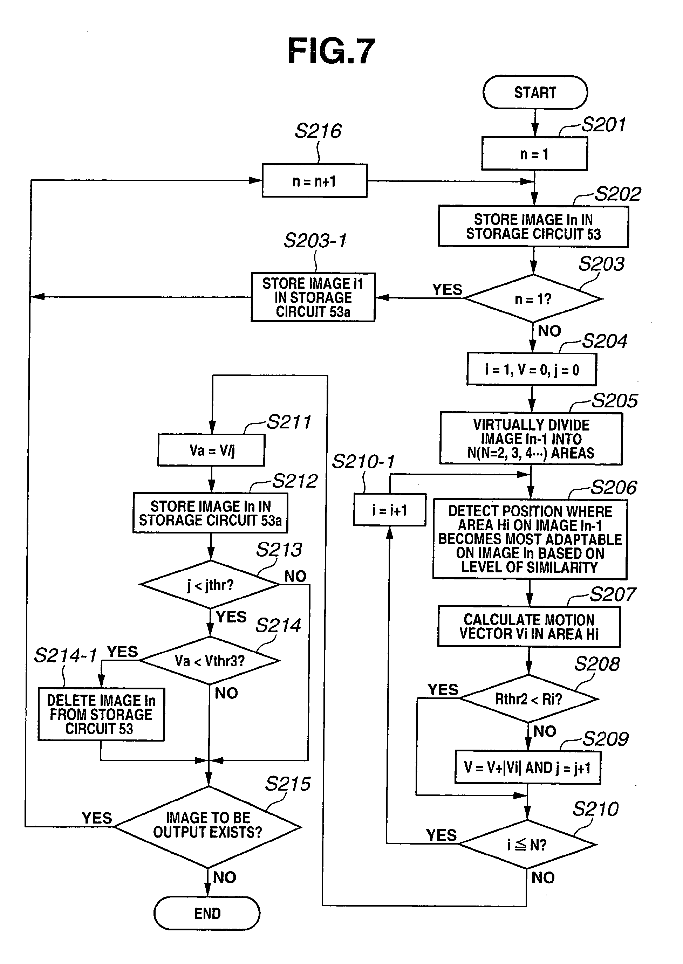

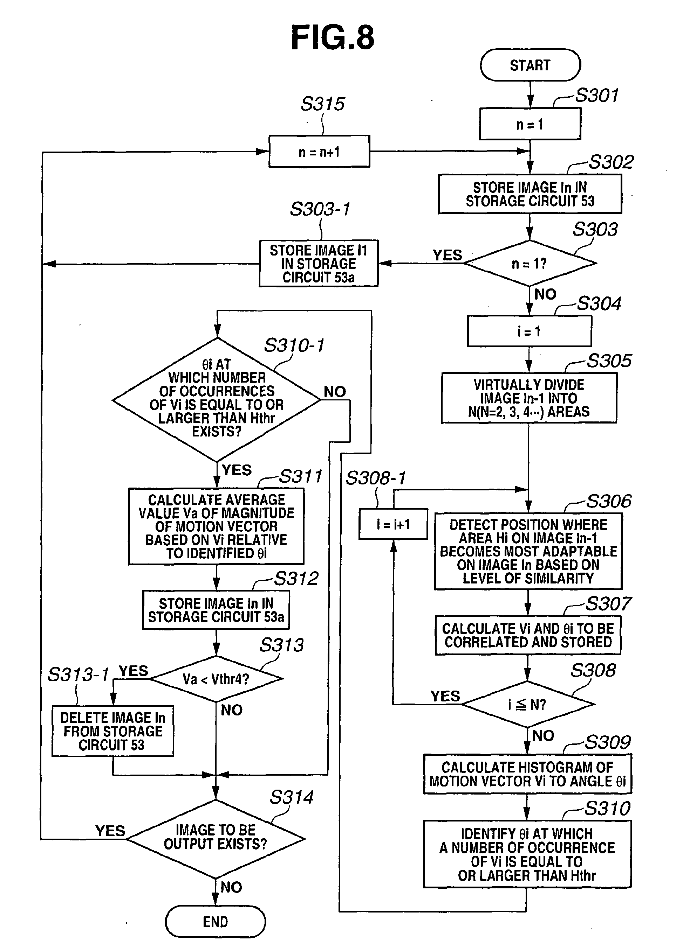

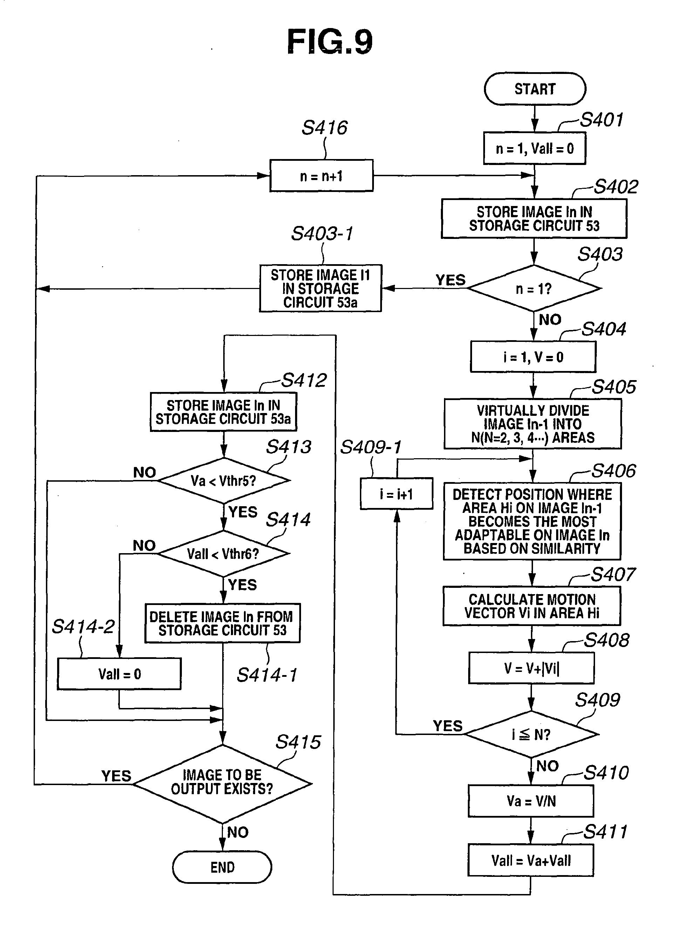

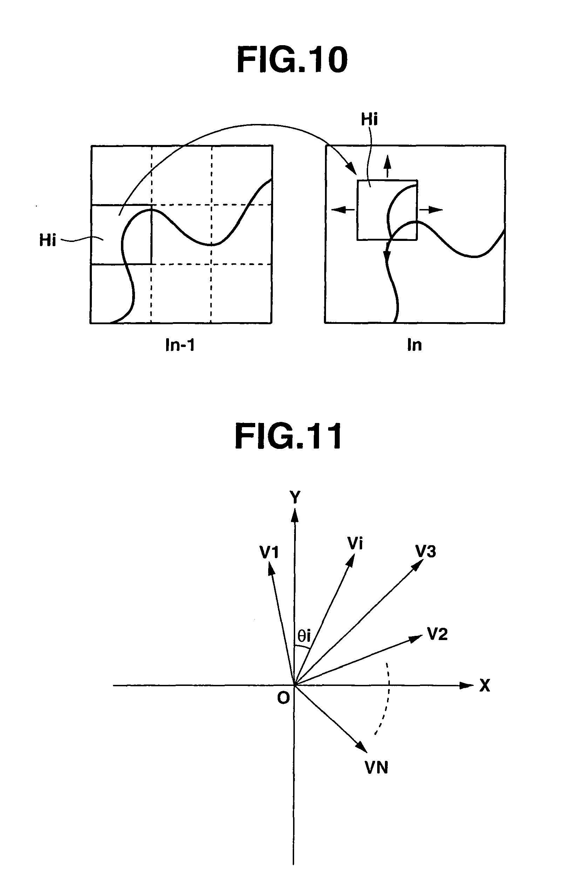

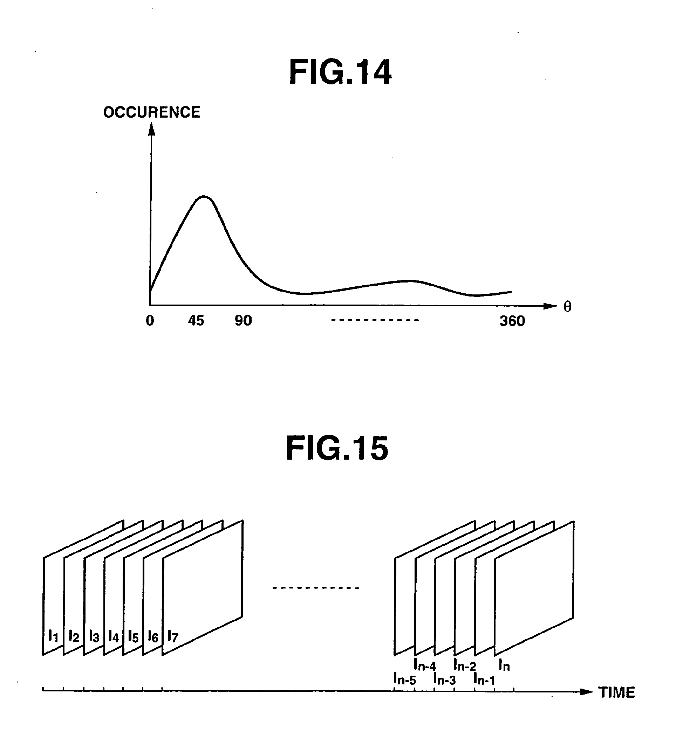

Fig. 1 is a view schematically showing a medical image processing system according to a first embodiment. Fig. 2 is a view showing an exemplary state where an external unit according to the first embodiment is connected to a terminal. Fig. 3 is a view showing an exemplary inner structure of a capsule medical device according to the first embodiment. Fig. 4 is a block diagram of each structure of the external unit and the terminal according to the first embodiment. Fig. 5 is a flowchart of an exemplary process, different from the one shown in Fig. 4, executed by the external unit according to the first embodiment with respect to image signals output from the capsule medical device. Fig. 6 is a flowchart of another exemplary process, different from those shown in Figs. 4 and 5, executed by the external unit according to the first embodiment with respect to image signals output from the capsule medical device. Fig. 7 is a flowchart of another exemplary process, different from those shown in Figs. 4, 5 and 6, executed by the external unit according to the first embodiment with respect to image signals output from the capsule medical device. Fig. 8 is a flowchart of another exemplary process, different from those shown in Figs. 4, 5, 6 and 7, executed by the external unit according to the first embodiment with respect to image signals output from the capsule medical device. Fig. 9 is a flowchart of another exemplary process, different from those shown in Figs. 4, 5, 6, 7 and 8, executed by the external unit according to the first embodiment with respect to image signals output from the capsule medical device. Fig. 10 is a view showing an exemplary process for detecting the position where an area of the image on a previous frame becomes most adaptable to the image on the current frame. Fig. 11 is a view graphically showing an angle based on a direction of a motion vector. Fig. 14 is a view showing an exemplary histogram of angles of the motion vectors.

As shown in Fig. 1, a medical image processing system 1 includes a capsule endoscope 3 as a capsule medical device which is inserted into the body of a patient 2 via the oral route for capturing images of the body cavity of the patient 2 and outputting the captured image of the body cavity of the patient 2 as an image signal, an antenna unit 4 externally attached to the patient 2 for wirelessly receiving the image signal output from the capsule endoscope 3, and an external unit 5 detachably connected to the antenna unit 4 via a cable and the like.

The antenna unit 4 includes a plurality of antennas 11 for receiving the image signals output from the capsule endoscope 3 on the surface of a jacket 10 worn by the patient 2 as shown in Fig. 1 and outputs the received image signals to the external unit 5 via the cable and the like. The antenna unit 4 may be of a skin patch type that allows direct application to the patient 2.

The external unit 5 serving as a medical control device has a box-like shape as shown in Fig. 1, for example, and is attached to a belt worn by the patient 2 with a detachable hook. A liquid crystal display monitor 12 that displays the state of the external unit 5 and the like and an operation switch 13 through which the operation commands are issued to the external unit 5 are provided on the front surface of the external unit 5. The external unit 5 is further structured to be detachably connected to a cradle 6 as shown in Fig. 2. The external unit 5 may only be provided with an alarm LED indicating the battery level, and a power switch and the like serving as the operation switch 13.

In the case where the capsule endoscope 3 is inserted into the body cavity of the patient 2 to capture the image thereof, the body cavity image of the patient 2 captured by the capsule endoscope 3 is output as the image signal, and the image signal is received by the antenna unit 4, and then output to the external unit 5 connected to the antenna unit 4 via the cable and the like. The external unit 5 stores image signals output from the antenna unit 4.

As shown in Fig. 2, a terminal 7 as a personal computer and the like includes a keyboard 8a and a mouse 8b through which operation commands are issued to various portions of the terminal 7, a monitor 8c serving as a display portion, and a terminal body 9 serving as the medical image processing device.

In the above-described structure, when the external unit 5 is connected to the terminal 7 via a cradle 6, the external unit 5 is electrically coupled with the terminal body 9 of the terminal 7. In such a state, the external unit 5 outputs the image signal stored therein to the terminal body 9 based on the operation command through the keyboard 8a and the mouse 8b, for example. The terminal body 9 stores the image signals output from the external unit 5 to be output to the monitor 8c based on the operation commands through the keyboard 8a and the mouse 8b.

As shown in Fig. 3, an exterior of the capsule endoscope 3 is formed of an exterior member 14 having a rear end of a cylindrical portion sealed and a dome type cover member 14a with a semispherical shape water tightly connected to a distal end side of the cylindrical portion with an adhesive to seal the distal end side of the cylindrical portion. Therefore, in the state where the exterior member 14 is connected to the dome type cover member 14a, the exterior of the capsule endoscope 3 has a water tight capsule configuration.

The portion covered with the transparent dome type cover member 14a and the portion adjacent thereto inside the capsule endoscope 3 are provided with an objective lens 15 that forms an image of the body cavity of the patient 2 incident through the dome type cover member 14a, a lens frame 16 for attaching the objective lens 15, an image pickup element 17 that forms an image pickup unit, and an LED 18 as shown in Fig. 3.

The image pickup element 17 as a CCD or the like is provided at the position where the objective lens 15 forms the image such that the body cavity image of the patient 2 within the observation field of the objective lens 15 is captured, and the captured body cavity image is output as the image pickup signal.

Four LEDs 18 are provided on the same plane around the objective lens 15, for example for irradiating white light rays for illumination to the body cavity of the patient 2.

The portion covered with the exterior member 14 and the portion adjacent thereto inside the capsule endoscope 3 are provided with a process circuit 19, a communication process circuit 20, batteries 21 each as a primary battery, for example, button battery for supplying voltage to the process circuit 19 and the communication process circuit 20 required to drive those circuits, and an antenna 23 which is connected to the communication process circuit 20 and allows the wireless communication of the various signals therebetween. The image pickup element 17, LEDs 18 and the like contained in the capsule endoscope 3 are arranged on the substrate (not shown). The respective substrates are connected to the corresponding flexible substrates.

The process circuit 19 is provided on the back surface of the image pickup element 17 and includes a CPU (not shown) and a memory that stores the program which allows the CPU to execute a predetermined process. The process circuit 19 that forms the image pickup unit drives the image pickup element 17 and the LEDs 18, and generates image signals based on the image pickup signal output from the image pickup element 17 so as to output the generated image signals. The process circuit 19 is further structured to control the image pickup element 17 with respect to the timing for capturing the body cavity images of two frames per second. The process circuit 19 as a control unit of the capsule endoscope 3 controls the image pickup timing of the image pickup element 17 and the light amount of the light irradiated from the LED 18 based on the signal such as operation command signal received from the external unit 5 via the antenna 23 and the communication process circuit 20. The process circuit 19 includes a selector circuit (not shown) by which the image pickup signals generated by the process circuit 19 are output while being selectively converted into the image signals. In other words, the process circuit 19 functions as the selector unit of the capsule endoscope 3.

The communication process circuit 20 processes, for example, modulates the image signal, and outputs the processed, that is, modulated signal to the antenna 23 such that the image signal output from the process circuit 19 is wirelessly transmitted via the antenna 23. Upon reception of the signal, for example, the operation command signal transmitted from the external unit 5 via the antenna 23, the communication process circuit 20 processes, for example, demodulates the signal such as the operation command signal, and outputs the processed, that is, demodulated signal to the process circuit 19.

As shown in Fig. 4, the external unit 5 includes a communication process circuit 51, a selector circuit 52, a storage circuit 53, a control circuit 54 and a communication process circuit 55 therein.

In the state where the external unit 5 is connected to the antenna unit 4, for the purpose of wirelessly transmitting the signal such as the operation command signal output from the control circuit 54 via the antenna unit 4, the communication process circuit 51 processes, for example, modulates the signal such as the operation command signal, and then outputs the processed, that is, modulated signal to the antenna unit 4. When receiving an image signal transmitted from the endoscope 3 via the antenna unit 4 in the state where the external unit 5 is connected to the antenna unit 4, the communication process circuit 51 processes, for example, demodulates the image signal, and then outputs the processed, that is, demodulated image signal to the selector circuit 52.

The selector circuit 52 serving as a selector unit of the external unit 5 outputs the image signal output from the communication process circuit 51 to the storage circuits 53 and 53a based on the control executed by the control circuit 54. The selector circuit 52 outputs the image signal stored in the storage circuit 53 to the control circuit 54 based on the control executed thereby.

The storage circuits 53 and 53a each serving as a storage unit of the external unit 5 store the image signal output from the selector circuit 52, and output the stored image signal to the selector circuit 52.

The control circuit 54 serving as a control unit of the external unit 5 includes a CPU (not shown) and a memory that stores the program which allows the CPU to execute a predetermined process, and controls the selector circuit 52 to select the image signal to be stored in the storage circuit 53, or the image signal to be read from the storage circuits 53 and 53a based on the preinstalled program. In the state where the external unit 5 is connected to the terminal body 9 via the cradle 6, the control circuit 54 controls such that the image signal read from the storage circuit 53 is output to the terminal body 9 via the communication process circuit 55 based on the operation command signal output through operation of the keyboard 8a and the mouse 8b connected to the terminal body 9. The control circuit 54 executes the control with respect to the respective portions of the capsule endoscope 3 and the external unit 5 and the processes relevant to such control based on the operation command signal output through the operation switch 13. In case of executing the aforementioned control and process relevant thereto, the control circuit 54 may be structured to retain the results of the control and the process temporarily in a resistor (not shown) and the like.

In the state where the external unit 5 is connected to the terminal body 9 via the cradle 6, the communication process circuit 55 outputs the signal, for example, operation command signal output through operation of the keyboard 8a and the mouse 8b connected to the terminal body 9 to the control circuit 54, and further outputs the image signal output from the control circuit 54 to the terminal body 9.

As shown in Fig. 4, the body 9 of the terminal 7 includes a communication process circuit 91, a storage circuit 92, a control circuit 93 and an image signal process circuit 94 therein.

In the state where the external unit 5 is connected to the terminal body 9 via the cradle 6, the communication process circuit 91 outputs the signal, for example, the operation command signal output through operation of the keyboard 8a and the mouse 8b to the external unit 5. The communication process circuit 91 outputs the image signal output from the external unit 5 to the storage circuit 92 based on the control executed by the control circuit 93.

The storage circuit 92 serving as the storage unit of the terminal body 9 stores the image signal output from the communication process circuit 91, and outputs the stored image signal to the control circuit 93.

The control circuit 93 serving as a control unit of the terminal body 9 reads the image signal stored in the storage circuit 92 based on the operation command signal output through operations of the keyboard 8a and the mouse 8b, and controls such that the read image signal is output to the image signal process circuit 94. In the case where the operation commands through the keyboard 8a and the mouse 8b are issued to the capsule endoscope 3 and the external unit 5, the control circuit 93 controls the operation command signal through the keyboard 8a and the mouse 8b to be output to the communication process circuit 91. The control circuit 93 serving as the selector unit of the terminal body 9 includes a CPU (not shown) and a memory that stores the program which allows the CPU to execute a predetermined process. The control circuit 93 further controls the storage circuit 92 and the image signal process circuit 94 such that selection between the image signal stored in the storage circuit 92 and the image signal to be output to the image signal process circuit 94 is made based on the preinstalled program.

The image signal process circuit 94 processes such that the image signal is displayed on the monitor 8c as the body cavity image based on the image signal output from the control circuit 93, and outputs the processed image signal to the monitor 8c.

Next, operations for the use of the medical image processing system 1 according to the embodiment will be described referring to Figs. 1 to 11. The image of the body cavity based on the image signal of the nth (n=1, 2, 3 ...) frame will be referred to as In hereinafter.

When the patient 2 swallows the capsule endoscope 3 through the mouth, the image pickup element 17 of the capsule endoscope 3 captures the body cavity image of the patient 2 in the observation field of the objective lens 15. The body cavity image thus captured is output as the image pickup signal. The process circuit 19 generates and outputs the image signal of the nth frame based on the image pickup signal output from the image pickup element 17. In the state immediately after inserting the capsule endoscope 3 through the mouth of the patient 2, the process circuit 19 is expected to output the image signal of the first frame (n=1). The process circuit 19 generates the image signals at every frame from the first frame sequentially to output the image signals.

The image signal of the nth frame is wirelessly output to the antenna unit 4 connected to the external unit 5 from the process circuit 19 via the communication process circuit 20 and the antenna 23. Upon reception of the image signal of the nth frame output from the capsule endoscope 3, the antenna unit 4 outputs the image signal of the nth frame to the external unit 5. The control circuit 54 of the external unit 5 controls the selector circuit 52 such that the image In based on the image signal of the nth frame output from the antenna unit 4 is stored in the storage circuit 53 in steps S1 and S2 shown in Fig. 5. When it is detected that the image signal output from the antenna unit 4 corresponds with that of the first frame (n=1) in step S3 of Fig. 5, the control circuit 54 controls the selector circuit 52 such that the image I1 based on the image signal of the first frame is stored in the storage circuit 53a in step S3-1 of Fig. 5.

When it is detected that the image signal output from the antenna unit 4 does not correspond with that of the first frame (n=1 ) in step S3 of Fig. 5, the control circuit 54 of the external unit 5 reads the image signal of the (n-1)th frame stored in the storage circuit 53a via the selector circuit 52, sets the retained value i to 1, and further sets the value V to 0 in step S4 of Fig. 5.

Then the control circuit 54 virtually divides the image In-1 of the body cavity based on the image signal of the (n-1)th frame into N (N=2, 3, 4, ...) areas set by the preinstalled program in step S5 of Fig. 5. In the present embodiment, the control circuit 54 executes the aforementioned process such that the image In-1 is divided into N (N=9 in Fig. 10) rectangular areas Hi (i=1, 2, ..., N).

Thereafter, the control circuit 54 detects the position where the area Hi on the image In-1 becomes most adaptable on the image In based on the level of similarity in step S6 of Fig. 5.

More specifically, the control circuit 54 performs template matching to set the area Hi on the image In-1 to the template area, and then obtains the level of similarity between the template area and a partial area Tj (j=1, 2, ..., M) on the image In having the same size as that of the template area as well as the position of the partial area Tj in which the level of similarity is the highest. The level of similarity R is the value calculated using formulae (1) to (3) based on the normalization cross-correlation as shown below.

$$R = \frac{\displaystyle\sum_{j=1}^{M}\sum_{i=1}^{N}\{H(i,j)-\mu_H\}\{T(i,j)-\mu_T\}}{\sqrt{\displaystyle\sum_{j=1}^{M}\sum_{i=1}^{N}\{H(i,j)-\mu_H\}^2\{T(i,j)-\mu_T\}^2}} \quad \cdots \quad (1)$$

$$\mu_H = \frac{1}{NM}\sum_{j=1}^{M}\sum_{i=1}^{N}H(i,j) \quad \cdots \quad (2)$$

$$\mu_T = \frac{1}{NM}\sum_{j=1}^{M}\sum_{i=1}^{N}T(i,j) \quad \cdots \quad (3)$$

In the formulae (1) to (3) shown above, H denotes the template area on the image In-1 with P x Q pixels, and T denotes the partial area on the image In with P x Q pixels. The level of similarity R in the above formula (1) is in the range of $-1 \leq R \leq 1$.

In step S7 of Fig. 5, the control circuit 54 calculates the motion vector Vi indicating the distance and direction that the area Hi moves from the position in the virtually divided state on the image In-1 to the position on the image In detected in step S6 of Fig. 5. Assuming that the center of the area Hi as the template area on the image In-1 is set to (xi, yi), and the center of the partial area Tj on the image In where the level of similarity to the area Hi becomes the highest is set to (xj, yj), the motion vector Vi may be calculated using the following formula (4).

$$Vi = (x_i - x_j, y_i - y_j) \quad \cdots \quad (4)$$

Furthermore, the control circuit 54 calculates the magnitude of the motion vector Vi, as the displacement value |Vi| based on the motion vector Vi, and adds the calculated value |Vi| to the value V as the sum of the motion vectors in step S8 of Fig. 5. The magnitude of the motion vector Vi, that is, |Vi| may be calculated using the following formula (5).

$$|Vi| = \sqrt{(x_i - x_j)^2 + (y_i - y_j)^2} \quad \cdots \quad (5)$$

The control circuit 54 adds each magnitude |Vi| of the motion vector Vi in the N areas Hi to the sum V of the magnitude of the motion vector while calculating the motion vector Vi and the magnitude |Vi| thereof in the N areas Hi in steps S6, S7, S8, S9 and S9-1 of Fig. 5.

Based on the sum V of the magnitude of the motion vectors to which magnitude values of the N motion vectors Vi from |V1| to |VN| are added, and the number N of the divided areas, the control circuit 54 calculates the value V/N, which is retained as the average value Va of the magnitude of the motion vector in step S10 of Fig. 5, and controls the selector circuit 52 to store the image In in the storage circuit 53a in step S11 of Fig. 5.

Then the control circuit 54 compares the average value Va of the magnitude of the motion vector with a threshold value Vthr1. If it is detected that the average value Va of the magnitude of the motion vector is smaller than the threshold value Vthr1 in step S12 of Fig. 5, the control circuit 54 determines that the level of similarity between the images In-1 and In is high, and controls the selector circuit 52 to delete the image In from the storage circuit 53 in step S12-1 of Fig. 5. Note that the threshold value Vthr1 corresponds to the average value Va of the magnitude of the motion vector, which may be a value set as a predetermined number of pixels, for example. If it is detected that the average value Va of the magnitude of the motion vector is equal to or larger than the threshold value Vthr1 in step S12 of Fig. 5, the control circuit 54 determines that the level of similarity between the images In-1 and In is relatively low, and continues the control routine without processing the image In.

When detecting that the image to be output further exists in step S 13 of Fig. 5, the control circuit 54 sets the value n to n+1 in step S 14 of Fig. 5, and executes the above-described process from steps S2 to S 13 shown in Fig. 5 with respect to the next image In + 1. When it is detected that the image to be output does not exist in step S 13 of Fig. 5, the control circuit 54 ends a series of the process.

The process and control executed by the respective portions of the external unit 5 are not limited to those described above, and may be executed based on the results of comparison between the level of similarity and the threshold value as described below, for example.

The control circuit 54 of the external unit 5 executes the same processes as those in the aforementioned steps S1, S2, S3 and S3-1 of Fig. 5 with respect to the image I1 based on the image signal of the first frame in steps S101, S102, S103 and S103-1 of Fig. 6. That is, the control circuit 54 controls the selector circuit 52 to store the image I1 in the storage circuits 53 and 53a.

When it is detected that the image signal output from the antenna unit 4 does not correspond with that of the first frame (n=1) in step S103 of Fig. 6, the control circuit 54 reads the image signal of the (n-1)th frame stored in the storage circuit 53a via the selector circuit 52, and sets retained values of i, V and j to 0, respectively in step S104 of Fig. 6.

Then the control circuit 54 executes the same processes as those in the aforementioned steps S5, S6 and S7 shown in Fig. 5 with respect to the body cavity image In-1 based on the image signal of the (n-1)th frame in step S105, S106 and S107 of Fig. 6. That is, the control circuit 54 divides the image In-1 into N rectangular areas Hi, and detects the position where the area Hi on the image In-1 becomes the most adaptable on the image In based on the level of similarity so as to calculate the level of similarity Ri at the detected position, and the motion vector Vi of the area Hi. The level of similarity Ri may be calculated using the formulae (1) to (3) as the value equivalent to the level of similarity R in the formula (1), for example.

The control circuit 54 further compares the level of similarity Ri with a threshold value Rthr. When it is detected that the value of the level of similarity Ri is equal to or smaller than the threshold value Rthr in step S108 of Fig. 6, the control circuit 54 determines that the level of similarity between the images In-1 and In is relatively low. Then the value $|Vi|$ is added to the value V as the sum of the magnitudes of the motion vectors in step S109 of Fig. 6. Besides the addition of the value $|Vi|$ to the value V, the control circuit 54 adds 1 to the value j which indicates the number of values $|Vi|$ equal to or smaller than the threshold value Rthr in step S109 of Fig. 6. The threshold value Rthr is set to a predetermined value conforming to the level of similarity between the area Hi of the image In-1 and the partial area Tj of the image In, for example, to satisfy the condition of $-1 \leq Rthr \leq 1$ in accordance with the level of similarity R in the above-described formula (1). In the aforementioned process, if it is detected that the value of the level of similarity Ri is larger than the threshold value Rthr in step S108 of Fig. 6, the control circuit 54 determines that the level of similarity between the images In-1 and In is high. The control circuit 54, thus continues the control routine without adding the value $|Vi|$ to the value V as the sum of the magnitudes of the motion vectors nor adding 1 to the value j.

The control circuit 54 calculates the motion vectors Vi and the levels of similarity Ri for the respective N areas Hi such that each of the calculated levels of similarity Ri is compared with the threshold value Rthr in steps S106, S107 and S108 of Fig. 6. Thereafter, the control circuit 54 selects only the value $|Vi|$ that conforms to the comparison result from those of the N areas Hi so as to be added to the value V, and further adds 1 to the value j that indicates the number of values $|Vi|$ conforming to the comparison result in steps S109, S 110 and S 110-1 of Fig. 6.

The control circuit 54 calculates the value V/j based on the sum of the magnitudes of the vectors V and the aforementioned value j so as to retain the calculated value as an average value Va of the magnitude of the motion vector in step S111 of Fig. 6, and controls the selector circuit 52 to store the image In in the storage circuit 53a in step S112 of Fig. 6.

The control circuit 54 compares the average value Va of the magnitude of the motion vector with a threshold value Vthr2. When detecting that the average value Va of the magnitude of the motion vector is smaller than the threshold value Vthr2 in step S 113 of Fig. 6, the control circuit 54 determines that the level of similarity between the images In-1 and In is high, and controls the selector circuit 52 to delete the image In from the storage circuit 53 in step S 113-1 of Fig. 6. The threshold value Vthr2 corresponds to the average value Va of the magnitudes of the motion vectors and is a value set as a predetermined number of pixels for example. When detecting that the average value Va of the magnitude of the motion vector is equal to or larger than the threshold value Vthr2 in step S 113 of Fig. 6, the control circuit 54 determines that the level of similarity between the images In-1 and the In is relatively low, and continues the control routine without processing the image In.

When detecting that the image to be output further exists in step S 114 of Fig. 6, the control circuit 54 sets the value n to n+1 in step S 115 of Fig. 6, and executes processes from steps S102 to S 114 of Fig. 6 as described above on the next image In+1. When detecting that the image to be output does not exist in step S114 of Fig. 6, the control circuit 54 ends the series of the process.

The process and control executed by the respective portions of the external unit 5 are not limited to those described above. The process and control may be executed based on the number of the values of magnitude of the motion vector conforming to the result of the comparison between the level of similarity and the threshold value as described below.

The control circuit 54 of the external unit 5 executes the same processes as those in aforementioned steps S1, S2, S3 and S3-1 of Fig. 5 with respect to the image I1 based on the image signal of the first frame in steps S201, S202, S203 and S203-1 of Fig. 7. Specifically, the control circuit 54 controls the selector circuit 52 to store the image I1 in the storage circuits 53 and 53a.

When detecting that the image signal output from the antenna unit 4 does not correspond with that of the first frame (n=1) in step S203 of Fig. 7, the control circuit 54 of the external unit 5 executes the same processes as the aforementioned steps S104, S105, S106 and S107 of Fig. 6 on the body cavity image In-1 based on the image signal of (n-1)th frame in steps S204, S205, S206 and S207 of Fig. 7. That is, the control circuit 54 sets the retained value i to 1 and values of V and j to 0, respectively. Thereafter, the control circuit 54 divides the image In-1 into N rectangular areas Hi and detects the position where the area Hi on the image In-1 becomes the most adaptable on the image In based on the level of similarity. The control circuit 54 further calculates the level of similarity Ri at the detected position so as to calculate the motion vector Vi of the area Hi. The level of similarity Ri may be calculated with the formulae (1) to (3) as the value equal to the level of similarity R in the formula (1) as described above, for example.

The control circuit 54 compares the level of similarity Ri with the threshold value Rthr2. When detecting that the value of the level of similarity Ri is equal to or smaller than the threshold value Rthr2 in step S208 of Fig. 7, the control circuit 54 determines that the level of similarity between the images In-1 and In is relatively low, and adds the value |Vi| to the value V as the sum of the magnitudes of the motion vectors in step S209 of Fig. 7. Besides the addition of the value |Vi| to the value V, the control circuit 54 adds 1 to the value j that indicates the number of the values |Vi| equal to or smaller than the threshold value Rthr2 in step S209 of Fig. 7. The threshold value Rthr2 is set to a predetermined value conforming to the similarity between the area Hi of the image In-1 and the partial area Tj of the image In, for example, in accordance with the level of similarity R in the aforementioned formula (1) to satisfy the condition of -1≤Rthr2≤1. In the aforementioned process, when detecting that the value of the level of similarity Ri is larger than the threshold value Rthr2 in step S208 of Fig. 7, the control circuit 54 determines that the level of similarity between the images In-1 and In is high, and continues the control routine without adding the value |Vi| to the value V as the sum of the magnitudes of the motion vectors nor adding 1 to the value j.

The control circuit 54 calculates each value of the motion vector Vi and the level of similarity Ri in the N areas Hi, respectively such that comparison between the calculated level of similarity Ri and the threshold value Rthr2 is made in steps S206, S207 and S208 of Fig. 7. Thereafter, the control circuit 54 selects only the value |Vi| conforming to the comparison result from those in the N areas Hi so as to be added to the value V, and adds 1 to the number of the values |Vi| conforming to the comparison result in steps S209, S210 and S210-1 of Fig. 7.

The control circuit 54 calculates the value V/j based on the value V as the sum of the magnitudes of the motion vectors and the aforementioned value j, and retains the calculated value as the average value Va of the magnitude of the motion vector in step S211 of Fig. 7. The control circuit 54 controls the selector circuit 52 to store the image In in the storage circuit 53a in step S212 of Fig. 7.

Then, the control circuit 54 compares the value j that indicates the number of values |Vi| conforming to the comparison results in step S208 of Fig. 7 described above with a threshold value jthr. When detecting that the value j is smaller than the threshold value jthr in step S213 of Fig. 7, the control circuit 54 further compares the average value Va of the magnitude of the motion vector with a threshold value Vthr3. When detecting that the average value Va of the magnitude of the motion vector is smaller than the threshold value Vthr3 in step S214 of Fig. 7, the control circuit 54 determines that the level of similarity between the images In-1 and In is high, and controls the selector circuit 52 to delete the image In from the storage circuit 53 in step S214-1 of Fig. 7. The threshold value Vthr3 corresponds to the average value Va of the magnitude of the motion vector, which may be a value set as a predetermined number of pixels, for example. When it is detected that the value j is equal to or larger than the threshold value jthr in step S213 of Fig. 7, or the value j is smaller than the threshold value jthr and the value Va is equal to or larger than the threshold value Vthr3 in steps S213 and S214 of Fig. 7, the control circuit 54 determines that the level of similarity between the images In-1 and In is relatively low, and continues the control routine without processing the image In.

When detecting that the image to be output further exists in step S215 of Fig. 7, the control circuit 54 sets the value n to n+1 in step S216 of Fig. 7, and processes the image In+1 in steps from S202 to S215 of Fig. 7 as described above. When detecting that the image to be output does not exist in step S215 of Fig. 7, the control circuit 54 ends the series of the process.

The process and control executed by the respective portions of the external unit 5 are not limited to those described above, but may be executed based on the angle formed by the motion vector and the histogram of such angle, for example.

The control circuit 54 of the external unit 5 executes the same process as those of aforementioned steps of S1, S2, S3 and S3-1 of Fig. 5 with respect to the image I1 based on the image signal of the first frame in steps S301, S302, S303 and S303-1 of Fig. 8. That is, the control circuit 54 controls the selector circuit 52 to store the image I1 in the storage circuits 53 and 53a.

When detecting that the image signal output from the antenna unit 4 does not correspond with that of the first frame (n=1) in step S303 of Fig. 8, the control circuit 54 reads the image signal of the (n-1)th frame stored in the storage circuit 53a, and sets the retained value i to 0 in step S304 of Fig. 8. The control circuit 54 virtually divides the body cavity images In-1 based on the image signal of the (n-1)th frame into N (N=2, 3, 4 ...) areas set in the preinstalled program in step S305 of Fig. 8.

Likewise the process in step S6 shown in Fig. 5, the control circuit 54 detects the position where the area Hi on the image In-1 becomes most adaptable on the image In based on the level of similarity in step S306 of Fig. 8. Based on the detection results, the control circuit 54 calculates the value of the motion vector Vi and the angle θi based on the direction of the motion vector Vi as graphically shown in Fig. 11 such that the calculated motion vector Vi and the angle θi based on the direction of the motion vector Vi are correlated and retained in step S307 of Fig. 8.

The control circuit 54 calculates the motion vectors Vi and the angles θi based on the direction of the motion vector Vi in the N areas Hi, respectively such that the calculated motion vector Vi and the angle θi based on the direction of the motion vector Vi are correlated and retained in steps S306, S307, S308 and S308-1 of Fig. 8.

Based on the N motion vectors Vi from V1 to VN, and N angle values θi from θ1 to θN which are retained in correlation therewith, the histogram of the motion vector Vi with respect to the angle θi is obtained in step S309 of Fig. 8. Based on the obtained histogram, the control circuit 54 identifies the angle θi at which the number of occurrence of the motion vector Vi has become equal to or larger than a threshold value Hthr in step S310 of Fig. 8.

When detecting that the angle θi at which the number of occurrence of the motion vector Vi is equal to or larger than the threshold value Hthr does not exist, the control circuit 54 determines that the level of similarity between the images In-1 and In is relatively low in step S310-1 of Fig. 8, and executes step S314 of Fig. 8 to be described later without processing the image In. When detecting that the angle θi at which the number of occurrence of the motion vector Vi is equal to or larger than the threshold value Hthr exists, the control circuit 54 determines that the level of similarity between the images In-1 and In is relatively high in step S310-1 of Fig. 8, and executes the process in step S311 of Fig. 8.

The control circuit 54 detects all the motion vectors Vi which relate to the identified angle θi, and calculates and retain the average value Va of the magnitude of the motion vector based on the detected motion vector Vi in step S311 of Fig. 8. The control circuit 54 further controls the selector circuit 52 to store the image In in the storage circuit 53a in step S312 of Fig. 8.

The control circuit 54 compares the average value Va of the magnitude of the motion vector with a threshold value Vthr4. When detecting that the average value Va of the magnitude of the motion vector is smaller than the threshold value Vthr4 in step S313 of Fig. 8, the control circuit 54 determines that the level of similarity between the images In-1 and In is high, and controls the selector circuit 52 to delete the image In from the storage circuit 53 in step S313-1 of Fig. 8. The threshold value Vthr4 corresponds with the average value Va of the magnitude of the motion vector, which may be a value set as a predetermined number of pixels, for example. When detecting that the average value Va of the magnitude of the motion vector is larger than the threshold value Vthr4 in step S313 of Fig. 8, the control circuit 54 determines that the level of similarity between the images In-1 and In is relatively low, and continues the control routine without processing the image In.

When detecting that the image to be output further exists in step S314 of Fig. 8, the control circuit 54 sets the value n to n+1 in step S315 of Fig. 8, and processes the next image In+1 in steps from S302 to S314 of Fig. 8 as described above. When detecting that the image to be output does not exist in step S314 of Fig. 8, the control circuit 54 ends the series of the process.

The process and control executed by the respective portions of the external unit 5 are not limited to those described above, but may be executed based on the average value of the magnitude of the motion vectors of the respective images.

The control circuit 54 of the external unit 5 controls the selector circuit 52 to execute the same processes as those in aforementioned steps S1, S2, S3 and S3-1 of Fig. 5 with respect to the image I1 based on the image signal of the first frame, that is, controls the selector circuit 52 to store the image I1 in the storage circuits 53 and 53a, and to set the value Vall to be described later to 0 in steps S401, S402, S403 and S403-1 of Fig. 9. When detecting that the image signal output from the antenna unit 4 does not correspond with that of the first frame (n=1) in step S403 of Fig. 9, the control circuit 54 reads the image signal of the (n-1)th frame stored in the storage circuit 53a via the selector circuit 52, sets the retained value i to 1, and further sets values of V and j to 0, respectively in step S404 of Fig. 9.

The control circuit 54 executes the same processes as those in aforementioned steps S5, S6, S7, S8, S9 and S9-1 of Fig. 5 with respect to the body cavity image In-1 based on the image signal of the (n-1)th frame in steps S405, S406, S407, S408, S409 and S409-1 of Fig. 9. That is, the control circuit 54 divides the image In-1 into N rectangular areas Hi, detects the position where each of the N areas Hi becomes most adaptable on the image In based on the level of similarity and further calculates the motion vector Vi.

The control circuit 54 calculates the value V/N using the value V as the sum of the magnitudes of the motion vectors to which the magnitudes of N motion vectors Vi from |V1| to |VN| are added, and the number N of the divided areas. The calculated value is retained as the average value Va of the magnitude of the motion vector in step S410 of Fig. 9, and controls the selector circuit 52 to store the image In in the storage circuit 53a in step S412 of Fig. 9 while retaining the value Vall as the sum of the average values Va obtained by adding the average value Va in step S411 of Fig. 9.

The control circuit 54 compares the average value Va of the magnitude of the motion vector with a threshold value Vthr5. When detecting that the average value Va of the magnitude of the motion vector is smaller than the threshold value Vthr5 in step S413 of Fig. 9, the control circuit 54 further compares the value Vall with a threshold value Vthr6. The threshold values Vthr5 and Vthr6 correspond with the values Va and Vall, respectively, and may be a value set as a predetermined number of pixels, for example.

Then when detecting that the value Vall as the sum of Va is smaller than the threshold value Vthr6 in step S414 of Fig. 9, the control circuit 54 determines that the image In is one of a group of several consecutive sheets of images, and the level of similarity with respect to the image In-1 is relatively high. Then the control circuit 54 controls the selector circuit 52 to delete the image In from the storage circuit 53 in step S414-1 of Fig. 9. When detecting that the value of Vall as the sum of the values Va is larger than the threshold value Vthr6 in step S414 of Fig. 9, the control circuit 54 determines that the level of similarity between the images In and In-1 is relatively low and sets the value Vall to 0 in step S414-2 of Fig. 9, and continues control routine without processing the image In.

When detecting that the average value Va of the magnitude of the motion vector is larger than the threshold value Vthr5 in step S413 of Fig. 9, the control circuit 54 determines that the level of similarity between the images In and In-1 is relatively low, and continues the control routine without processing the image In.

When detecting that the image to be output further exists in step S415 of Fig. 9, the control circuit 54 sets the value n to n+1 in step S416 of Fig. 9 and executes steps from S402 to S415 of Fig. 9 as described above with respect to the next image In+1. When detecting that the image to be output does not exist in step S415 of Fig. 9, the control circuit 54 ends the series of the process.

As the external unit 5 executes the aforementioned processes with respect to the image based on the image signal output from the capsule endoscope 3 disposed in the body cavity of the patient 2, the image with high similarity to that of the previous frame, that is, the image with small fluctuation of the capsule endoscope 3 in the body cavity, which is regarded as the identical image through observation of the operator is deleted. At the same time, the image with low similarity to that of the previous frame, in other words, the image with great fluctuation of the capsule endoscope 3 in the body cavity, which is required to be observed by the operator can be stored.

The external unit 5 executes the aforementioned processes with respect to all the images based on the image signals stored in the storage circuit 53 to allow storage of only the image with great fluctuation of the capsule endoscope 3 in the body cavity as the minimum portion required for the operator to observe. The external unit 5 is capable of reducing the size of image signal data stored in the storage circuit 53 compared with the generally employed system.

The aforementioned processes shown in the flowcharts from Figs. 5 to 9 may be executed not only by the control circuit 54 of the external unit 5 but also by the control circuit 93 installed in the body 9 of the terminal 7 with respect to all the images based on the image signals stored in the storage circuit 53 of the external unit 5.

In the above case, when the external unit 5 is connected to the cradle 6, the control circuit 93 controls the communication process circuit 91 to read all the images based on the image signals stored in the storage circuit 53.

The control circuit 93 executes at least one of the processes in the flowcharts from Figs. 5 to 9 with respect to the image based on the image signal output from the external unit 5 connected to the cradle 6 so as to control the communication process circuit 91 and the storage circuit 92 not to store the image with high similarity to that of the previous frame, and to store the image with low similarity to that of the previous frame.

The control circuit 93 may be structured not only to execute the control and process for determining the images to be stored in the storage circuit 92 but also to execute the control and processes as described below.

The control circuit 93 controls the communication process circuit 91 and the storage circuit 92 to store all the images based on the image signals stored in the storage circuit 53 of the external unit 5 when the external unit 5 is connected to the cradle 6. The control circuit 93 executes one of the processes in the flowcharts from Figs. 5 to 9 with respect to all the images based on the image signals stored in the storage circuit 92. This makes it possible to store only the image with great fluctuation of the capsule endoscope 3 in the body cavity among all the aforementioned images as the minimum image portion required for the operator to observe. Accordingly, the terminal body 9 is allowed to reduce the data size of the image signal stored in the storage circuit 92 compared with the generally employed system.

In the case where one of the processes of the flowcharts from Figs. 5 to 9 is executed, the control circuit 93 may be structured to perform the control and process not only for determining the image to be stored in the storage circuit 92 but also for determining whether or not the image is displayed on the monitor 8c, for example.

In the aforementioned case, when the external unit 5 is connected to the cradle 6, the control circuit 93 controls the communication process circuit 91 to read all the images based on the image signals stored in the storage circuit 53.

The control circuit 93 then executes one of the processes in the flowcharts from Figs. 5 to 9 with respect to the image based on the image signal output from the external unit 5 connected to the cradle 6 to control the storage circuit 92 and the image signal process circuit 94 not to display the image with high similarity to that of the previous frame on the monitor 8c, and to display the image with low similarity to that of the previous frame on the monitor 8c, for example.

Besides the control and process for determining the image to be displayed on the monitor 8c, the control circuit 93 may be structured to perform the control and process as described below.

When the external unit 5 is connected to the cradle 6, the control circuit 93 controls the communication process circuit 91 and the storage circuit 92 to store all the images based on the image signals stored in the storage circuit 53 of the external unit 5. Then the control circuit 93 executes one of the processes of the flowcharts from Figs. 5 to 9 with respect to all the images based on the image signals stored in the storage circuit 92 to control the storage circuit 92 and the image signal process circuit 94 to display only the image with the great fluctuation of the capsule endoscope 3 in the body cavity among all the images as the minimum portion required for the operator to observe. This allows the operator to observe only the image of the captured portion required for the observation among those of the body cavity of the patient 2 captured by the capsule endoscope 3 while looking at the monitor 8c.

The processes of the flowcharts from Figs. 5 to 9 may be executed not only by the control circuit 54 of the external unit 5 but also by a process circuit 19 of the capsule endoscope 3 or a selector circuit (not shown) of the process circuit 19.

In the aforementioned case, the process circuit 19 executes one of the processes of the flowcharts from Figs. 5 to 9 with respect to the image pickup signal output from the image pickup element 17 such that the image pickup signal of the image with high similarity to that of the previous frame is not converted into the image signal, and the process is executed for converting the image pickup signal of the image signal with low similarity to that of the previous frame into an image signal to output the image signal to the communication process circuit 20.

As described above, the medical image processing system 1 according to the embodiment is structured to output the minimum image signal required for the operator to observe the live body based on the similarity between image signals of two or more frames. As a result, the medical image processing system 1 according to the present embodiment allows the operator to observe the live body with less burden compared with the generally employed case.

(Second Embodiment)

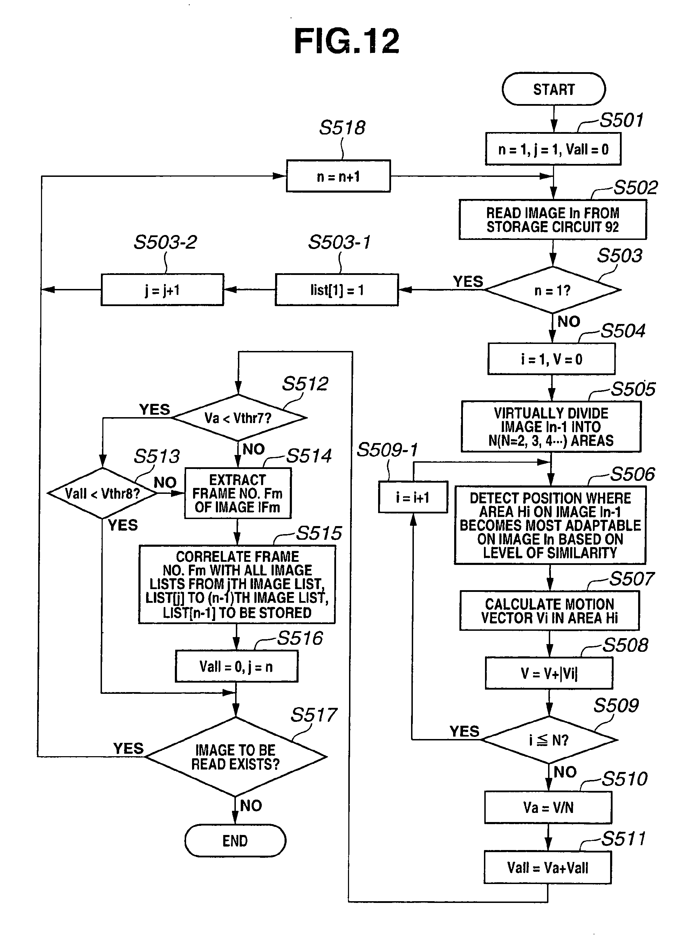

Figs. 12, 15, 16, 17 and 18 relate to a second embodiment of the preset invention. Note that, in the flowchart in Fig. 12, detailed explanation with respect to the same control and process contents as those in the first embodiment will be omitted. The same components as those of the first embodiment will be designated with the same reference numerals, and explanations thereof, thus will be omitted. The structure of the medical image processing system 1 according to the embodiment is the same as that of the first embodiment.

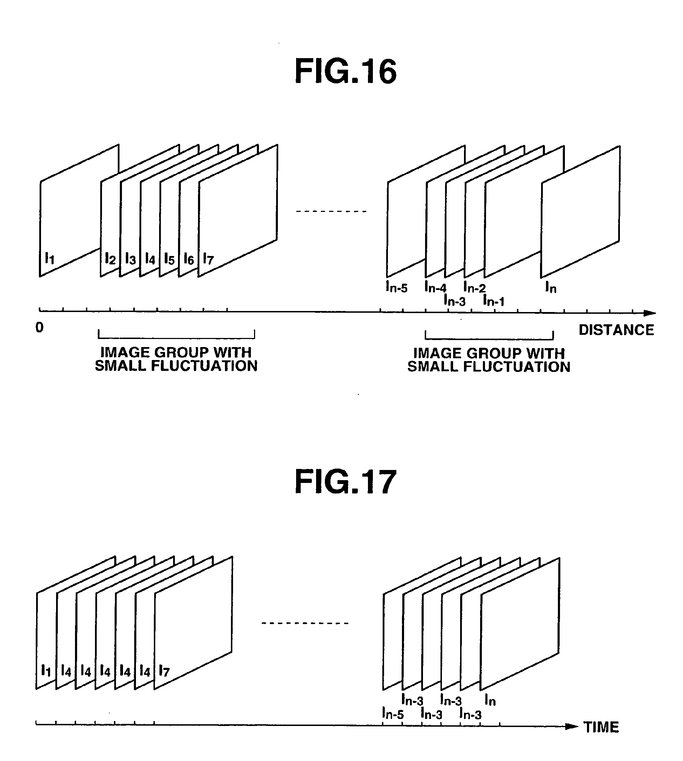

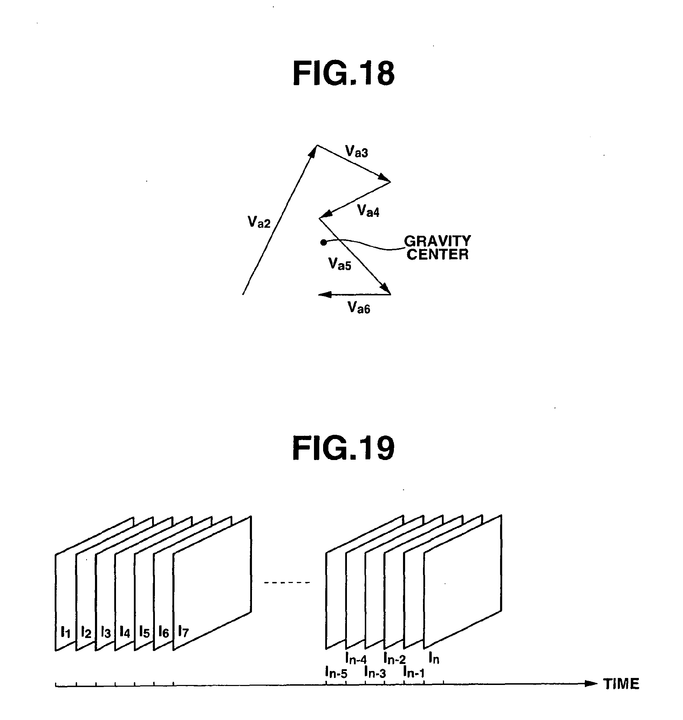

Fig. 12 is an exemplary flowchart for processing the image signal stored in the storage circuit by a terminal according to the second embodiment. Fig. 15 shows an example of the images displayed on the monitor in the case where the process shown in Fig. 12 is not executed. Fig. 16 graphically shows a group of images with small fluctuation among captured images in the process shown in Fig. 12. Fig. 17 shows the exemplary image displayed on the monitor when the process shown in Fig. 12 is executed. Fig. 18 shows the state where the motion vectors are connected with one another.

When the external unit 5 is connected to the cradle 6, the control circuit 93 installed in the body 9 of the terminal 7 controls the communication process circuit 91 and the storage circuit 92 to read and store all the images based on the image signals stored in the storage circuit 53.

The control circuit 93 executes steps from S501 to S511 of Fig. 12 substantially the same as steps S401 to S411 of Fig. 9 described in the first embodiment while reading the image stored in the storage circuit 92 one by one. Further, the control circuit 93 sets the value n to n+1 as shown in step S518 of Fig. 12 as substantially the same process as step S416 of Fig. 9. Simultaneously with the aforementioned process, the control circuit 93 sets the variable j (j=1, 2, 3 ...) that indicates the number of the image list [j] to 1 as the process required for reading the first image I1 in step S501 of Fig. 12. In case of the image I1 of the first frame, the list [1] as the first image list is correlated with 1 as the frame number of the image I1. That is, in step S503-1 of Fig. 12, the list [1] is set to 1 to be retained, and the process proceeds to step S503-2 of Fig. 12 where 1 is added to the variable j.

The control circuit 93 compares the average value Va of the magnitude of the motion vector with a threshold value Vthr7. When detecting that the average value Va of the magnitude of the motion vector is smaller than the threshold value Vthr7 in step S512 of Fig. 12, the control circuit 93 further compares the value Vall with a threshold value Vthr8. The threshold values Vthr7 and Vthr8 corresponding to the values Va and Vall, respectively may be a value set as a predetermined number of pixels.

When detecting that the value Vall is smaller than the threshold value Vthr8 in step S513 of Fig. 12, the control circuit 93 extracts the image In as the candidate of the image group that contains the image In-1, and executes the process shown in step S517 of Fig. 12 to be described later. In other words, when the process shown in step S513 of Fig. 12 is executed by the control circuit 93, the image In becomes the candidate of the image group that contains the image In-1.

When detecting that the average value Va of the magnitude of the motion vector is larger than the threshold value Vthr7 in step S512 of Fig. 12, or detecting that the value Va is smaller than the threshold value Vthr7, and the value Vall is larger than the threshold value Vthr8 in steps S512 and S513 of Fig. 12, the control circuit 93 determines that the image In cannot be added to the image group that contains the image In-1.

The control circuit 93 identifies an image IFm at an intermediate time point of a plurality of images from Ij to In-1 contained in the image group including the image In-1, and extracts a frame number Fm of the identified image IFm in step S514 of Fig. 12. The control circuit 93 controls the storage circuit 92 and the image signal process circuit 94 to correlate all the image lists from the jth image list (list[j]) to the (n-1)th image list (list[n-1]) with the frame number Fm so as to be stored in step S515 of Fig. 12.

The control circuit 93 sets the value of the retained Vall to 0, and the value of the variable j to n in step S516 of Fig. 12.

The control circuit 93 may be structured not only to extract an image at an intermediate time point of a plurality of images contained in the image group such that the frame number is stored in the storage circuit 92 but also to extract an image at the first or the last time point such that the frame number is stored in the storage circuit 92.

When detecting that the image to be read from the storage circuit 92 further exists in step S517 of Fig. 12, the control circuit 93 sets the value n to n+1 in step S518 of Fig. 12, and executes the aforementioned process from steps S502 to S517 with respect to the next image In+1. In step S517 of Fig. 2, when detecting that the image to be read from the storage circuit 92 does not exist, the control circuit 93 ends the series of the process.

In the case where the process of Fig. 12 has not been executed, the control circuit 93 controls the respective portions of the terminal body 9 to display all the captured images at set intervals as shown in Fig. 15. In the case where the process of Fig. 12 has been executed, the control circuit 93 controls the respective portions of the terminal body 9 to display a predetermined image on the monitor 8c based on the image list (list[j]) with the frame number of the image stored through the process of Fig. 12. In other words, the control circuit 93 controls the respective portions of the terminal body 9 to display the image with the frame number stored in correlation with the image list (list[j]) on the monitor 8c sequentially at predetermined intervals.

Specifically, in the case where the aforementioned process of Fig. 12 has been executed, the control circuit 93 combines images with small fluctuation between adjacent images among n images from I1 to In, that is, the adjacent images each having substantially the same scene into one image group as shown in Fig. 16. The control circuit 93 extracts among the images include in an image group an image that locates at substantially an intermediate time point of the image group, and controls the respective portions of the terminal body 9 to display the extracted image on the monitor 8c by the number of sheets of the images contained in the image group as shown in Fig. 17.

The control circuit 93 may be structured not only to extract the image at substantially the intermediate time point of the image group through the aforementioned process but also to extract the image based on the image list stored in the storage circuit 92 in the processes from steps S512 to S515 of Fig. 12 as described below.

When detecting that the average value Va of the magnitude of the motion vector is larger than the threshold value Vthr7, or the value Va is smaller than the threshold value Vthr7 and the value Vall is larger than the threshold value Vthr8, the control circuit 93 determines that the image In cannot be added to the image group that contains the image In-1. The control circuit 93 then calculates the composite motion vector Vc based on the average motion vector Vai (i=j, j+1, ..., n-1) corresponding to the respective images contained in the image group including the image In-1. Note that the composite motion vector Vc is expressed by the following formula (6).

$$V_c = V_{aj} + V_{aj+1} + ... + V_{an-1} \quad \cdot \cdot \cdot \quad (6)$$

The control circuit 93 performs the process for connecting the end point and the origin of the two motion vectors at adjacent time points with respect to the average motion vector Vai between the respective images contained in the image group. Thereafter, the control circuit 93 detects the gravity center of each of the origins of the motion vectors Vai.

The control circuit 93 then detects the position of the origin that is the closest to the gravity center, and the frame number i of the image corresponding to the motion vector Vai with the detected origin. The control circuit 93 controls the storage circuit 92 and the image signal process circuit 94 to store all the image lists from the jth image list (list [j]) to the (n-1)th image list (list[n-1]) in correlation with the frame number i.

More specifically, assuming that images from 12 to 17 are combined into one image group as shown in Fig. 16, the control circuit 93 calculates the average motion vectors Vai (i=2, 3, 4, 5 and 6) among the respective images, and connects end points and origins of the vectors at adjacent time points (for example, the end point of Va2 is connected to the origin of Va3) so as to connect all the average motion vectors Vai. Then the control circuit 93 calculates the gravity center based on the origins of the connected average motion vectors from Va2 to Va6 to detect the average motion vector Vai having the origin that is the closest to the calculated gravity center.

If the average motion vectors from Va2 to Va6 are connected as shown in Fig. 18, the control circuit 93 controls the storage circuit 92 and the image signal process circuit 94 to store all the image lists from the second image list (list [2]) to the sixth image list (list[6]) in correlation with the frame number 5 of the image 15 corresponding to the average motion vector Va5 with the origin that is the closest to the gravity center.

As the control circuit 93 executes the process of Fig. 12 and the control for displaying the image as shown in Fig. 17 as described above, the terminal 7 displays an image among a plurality of images contained in the image group with small fluctuation of the capsule endoscope 3 in the body cavity in addition to the image as the minimum portion required for the operator to observe.

Based on the similarity among images of two or more frames, the medical image processing system 1 according to the present embodiment is designed to display the image of the portion with great fluctuation of the capsule endoscope 3 in the body cavity, and to extract only an image from the image group with small fluctuation of the capsule endoscope 3 in the body cavity so as to be displayed on the monitor 8c. Accordingly, the medical image processing system 1 according to the present embodiment reduces the burden of the operator to observe the live body compared with the generally employed system.

(Third Embodiment)

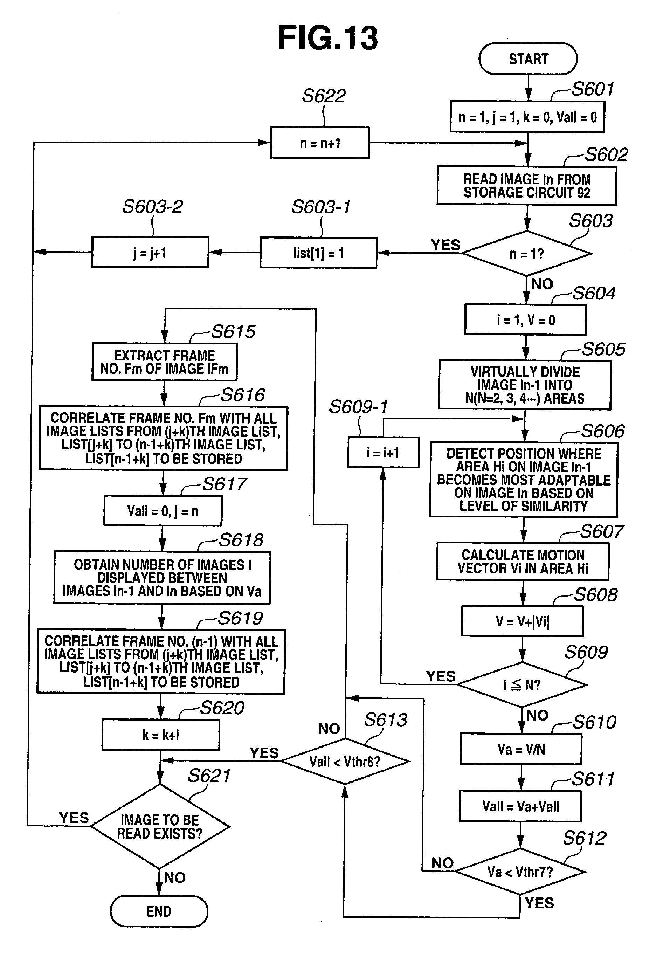

Figs. 13, 19, 20 and 21 relate to a third embodiment of the present invention. Note that in the flowchart shown in Fig. 13 the detailed explanation of the part of the same process and control contents as those of the first and the second embodiments will be omitted. The same components as those of the first and the second embodiments will be designated with the same reference numerals and explanations thereof, thus will be omitted. The medical image processing system 1 according to the embodiment is the same as that of the first and the second embodiments.

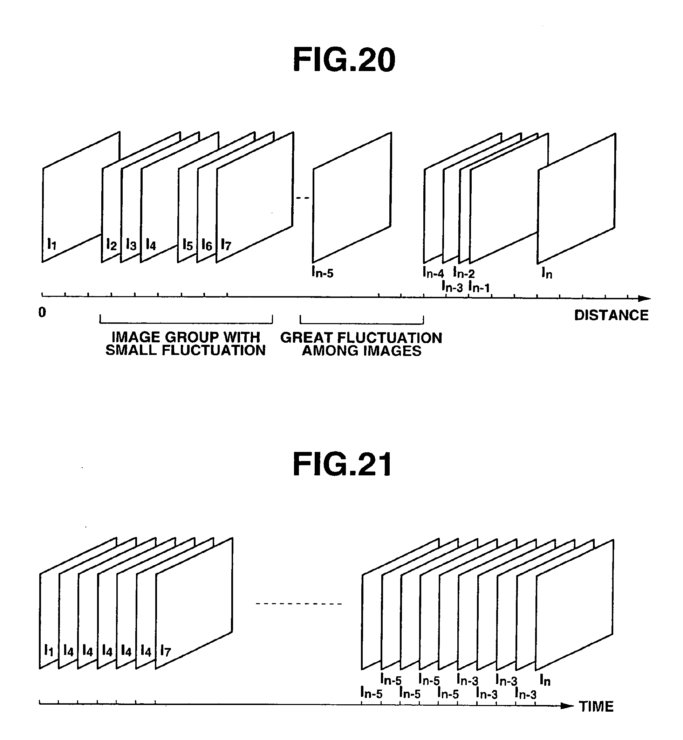

Fig. 13 is a flowchart representing an exemplary process executed by the terminal according to the third embodiment with respect to the image signal stored in the storage circuit. Fig. 19 shows an example of the images displayed on the monitor in the case where the process shown in Fig. 13 is not executed. Fig. 20 graphically shows an exemplary process of the group of images with small fluctuation among the captured images as an image group. Fig. 21 shows an example of the image displayed on the monitor in the case where the process shown in Fig. 13 has been executed.

When the external unit 5 is connected to the cradle 6, the control circuit 93 installed in the body 9 of the terminal 7 controls the communication process circuit 91 and the storage circuit 92 to read and store all the images based on the image signals stored in the storage circuit 53.

The control circuit 93 executes the processes from steps S601 to S611 of Fig. 13 which is substantially the same as those from steps S501 to S511 of Fig. 12 as described in the second embodiment while reading the images stored in the storage circuit 92 one by one. The control circuit 93 executes the process of step S622 of Fig. 13 which is substantially the same as that of step S518 of Fig. 12. The control circuit 93 sets the variable k indicating the total number of the images to be additionally displayed simultaneously with the process in step S601 of Fig. 13 as described above.

The control circuit 93 compares the average value Va of the magnitudes of the motion vectors with the threshold value Vthr7. When detecting that the average value Va of the magnitudes of the motion vectors is smaller than the threshold value Vthr7 in step S612 of Fig. 13, the control circuit 93 further compares the value Vall with the threshold value Vthr8. The threshold values 7 and 8 that correspond with values of Va and Vall, respectively may be a value set as a predetermined number of pixels, for example.

When detecting that the value Vall is smaller than the threshold value Vthr8 in step S613 of Fig. 13, the control circuit 93 extracts the image In as the candidate for the image group that contains the image In-1, and executes step S621 of Fig. 13 to be described below. In other words, when step S613 of Fig. 13 is executed by the control circuit 93, the image In becomes the candidate to be added to the image group that contains the image In-1.

When detecting that the average value Va of the magnitude of the motion vector is larger than the threshold value Vthr7 in step S612 of Fig. 13, or the value Va is smaller than the threshold value Vthr7 and the value Vall is larger than the threshold value Vthr8 in steps S612 and S613 of Fig. 13, the control circuit 93 determines that the image In cannot be added to the image group that contains the image In-1.

The control circuit 93 extracts the image IFm at substantially the intermediate time point of a plurality of images from the images Ij to In-1 of the image group including In-1, and the frame number Fm of the extracted IFm in step S615 of Fig. 13. The control circuit 93 controls the storage circuit 92 and the image signal process circuit 94 in step S616 of Fig. 13 to store all the image lists from the (j+k)th image list (list[j+k]) to the (n-1+k)th image list (list[n-1+k]) in correlation with the frame number Fm.

The control circuit 93 sets the retained value Vall to 0, and sets the variable j to the value n in step S617 of Fig. 13.

The control circuit 93 may be structured not only to extract the image at substantially the intermediate time point of a plurality of images contained in the image group such that the frame number is stored in the storage circuit 92 but also to extract the image at the first or the last time point of a plurality of images contained in the image group such that the frame number is stored in the storage circuit 92.

The control circuit 93 calculates the variable I that indicates the number of sheets of the image In-1 to be inserted between the images In-1 and In based on the average value Va of the magnitude of the motion vector therebetween in step S618 of Fig. 13. More specifically, assuming that the average value Va of the magnitude of the motion vector is set to 20 pixels, the control circuit 93 inserts one sheet of the image In-1 at every four pixels. That is, the control circuit 93 controls such that five sheets of the image In-1 are inserted between the images In-1 and In. In the case where the fluctuation between the images In-1 and In is excessively great, that is, the average value Va of the magnitude of the motion vector is equal to or larger than a predetermined value, the control circuit 93 executes the control to insert a predetermined number of sheets of the image In-1, for example, ten sheets between the images In-1 and In.

The control circuit 93 controls the storage circuit 92 and the image signal process circuit 94 to store all the image lists from the (j+k)th image list (list[j+k]) to the (j+k+1)th image list (list[j+k+1]) in correlation with the frame number (n-1) of the image In-1 in step S619 of Fig. 13, and executes the process for adding a variable 1 to the variable k in step S620 of Fig. 13.

When detecting that the image to be read from the storage circuit 92 further exists in step S621 of Fig. 13, the control circuit 93 sets the value n to n+1 in step S622 of Fig. 13 and executes the processes in aforementioned steps from S602 to S621 of Fig. 13 with respect to the next image In+1. In step S621 of Fig. 13, when detecting that the image to be read from the storage circuit 92 does not exist, the control circuit 93 ends the series of the process.

In the case where the aforementioned process shown in Fig. 13 has not been executed, the control circuit 93 controls the respective portions of the terminal body 9 to display all the captured images at set intervals as shown in Fig. 19. In the case where the aforementioned process shown in Fig. 13 has been executed, the control circuit 93 controls the respective portions of the terminal body 9 to display the predetermined image on the monitor 8c based on the image list (list[j]) with the image frame number stored through the aforementioned process of Fig. 13. In other words, the control circuit 93 controls the respective portions of the terminal body 9 to display the image of the frame number stored in correlation with the image list (list[j]) on the monitor 8c sequentially at predetermined intervals.

More specifically, in the case where the aforementioned process shown in Fig. 13 has been executed, the control circuit 93 combines a group of images with small fluctuation between adjacent images among n sheets of images from I1 to In, that is, the image group each having similar scene into one image group as shown in Fig. 20. Meanwhile, the control circuit 93 detects the portion with great fluctuation between two consecutive images at adjacent time points among the n sheets of images from I1 to In as shown in Fig. 20.

The control circuit 93 extracts an image at substantially the intermediate time point of images contained in the image group as shown in Fig. 21, and controls the respective portions of the terminal body 9 to display the extracted image by the number corresponding to the sheets of the image contained in the image group. As shown in Fig. 21, the control circuit 93 controls the respective portions of the terminal body 9 to insert the predetermined number of sheets of the image of a frame at a previous time point among those of two frames between the other images of the frame at the subsequent time point in the portion with the great fluctuation between images of two frames at adjacent time points so as to be displayed on the monitor 8c.

As the control circuit 93 executes the process shown in Fig. 13 and the control to display the image as shown in Fig. 21, the terminal 7 displays an image among a plurality of images contained in the image group having a portion with small fluctuation of the capsule endoscope 3 in the body cavity on the monitor 8c in addition to the image of the minimum portion required for the operator to observe the live body. As the control circuit 93 executes the process as shown in Fig. 13, the terminal 7 in the case where the level of similarity between those images is excessively low or no similarity exists in the image of two frames at consecutive time points inserts a predetermined number of sheets of the image of the frame at a previous time point between the other images of the frame at the subsequent time point.

Based on the similarity between images of two or more frames, the medical image processing system 1 according to the embodiment is structured to display the image of the portion with great fluctuation of the capsule endoscope 3 in the body cavity, and to extract only one image from the image group with small fluctuation of the capsule endoscope 3 in the body cavity so as to be displayed on the monitor 8c. Based on the similarity between images of two frames at consecutive time points, the medical image processing system 1 according to the embodiment is structured to display the images of two frames while supplementing the period therebetween in the case where those images of the two frames correspond with the image with great fluctuation of the capsule endoscope 3 in the body cavity. This allows the medical image processing system 1 according to the embodiment to reduce the burden of the operator to observe the live body compared with the generally employed system.

It is to be easily understood that the present invention is not limited to the aforementioned embodiments but may be modified or applied into various forms without departing from the scope of the present invention.

This application claims priority from Japanese Patent Application No. 2006-014444 filed on January 23, 2006. The contents of the disclosure are cited in the specification of the present application, claims, and the drawings alike.

**Claims**

1. A capsule medical device comprising:

   an image pickup unit that captures an image of a subject, and outputs the image of the subject as an image pickup signal; and
   a control unit that determines with respect to similarity among a plurality of images contained in an image group in accordance with the image pickup signals of two or more frames among those output from the image pickup unit based on a predetermined threshold value indicating a magnitude of fluctuation between the images contained in the image group, and controls to output an image signal based on a result of the determination.

2. The capsule medical device according to Claim 1, wherein the control unit determines with respect to the similarity between adjacent images of those contained in the image group based on the predetermined threshold value, and extracts the image pickup signal of one frame from the image pickup signals of the two or more consecutive frames based on the result of the determination so as to be output.

3. The capsule medical device according to Claim 1 or 2, wherein the predetermined threshold value corresponds with an average value of a displacement value that indicates a displacement amount of a portion of the image at a previous time point in a portion of the other image at a subsequent time point among the images contained in the image group.

4. The capsule medical device according to any one of Claims 1 to 3, wherein the image pickup unit includes a selector portion that selectively converts the image pickup signal into an image signal so as to be output.

5. The capsule medical device according to any one of Claims 1 to 3, wherein the control unit determines with respect to the similarity between the images contained in the image group by making a determination of a threshold value based on a similarity value that indicates the similarity between a portion of the image at the previous time point and a portion of the other image at the subsequent time point in the images contained in the image group.

6. The capsule medical device according to any one of Claims 1 to 3, wherein the control unit divides the image contained in the image group into a plurality of areas to calculate a motion vector and an angle formed by the motion vector in each of the divided areas, and determines with respect to the similarity between the images contained in the image group by making a determination of the threshold value based on a number of occurrences of the motion vector in a histogram of the angle.

7. The capsule medical device according to any one of Claims 1 to 3, wherein the control unit determines with respect to the similarity between the images contained in the image group by making a determination of a threshold value based on an average value of magnitudes of the motion vectors of the respective images contained in the image group.

8. A medical control device comprising:

   a selector unit for selectively outputting an image signal output from a medical device equipped with an image pickup unit that outputs a captured subject image as the image signal; and
   a control unit for determining with respect to a similarity between images among a plurality of images contained in an image group in accordance with the image signals of two or more frames among image signals output from the medical device based on a predetermined threshold value indicating fluctuation between the images contained in the image group, and controls to allow the selector unit to output the image signal based on a result of the determination.

**9.** The medical control device according to Claim 8, wherein the control unit determines with respect to the similarity between adjacent images of the images contained in the image group based on the predetermined threshold value, and controls to extract the image signal of one frame from the image signals of the two or more consecutive frames based on the result of the determination so as to allow the selector unit to output the extracted image signal.

**10.** The medical control device according to Claim 8 or 9, wherein the predetermined threshold value corresponds with an average value of a displacement value that indicates a displacement amount of a portion of the image at a previous time point in a portion of the other image at a subsequent time point among the images contained in the image group.

**11.** The medical control device according to any one of Claims 8 to 10, further comprising a storage unit for storing the image signal output from the image pickup unit, wherein the control unit executes the predetermined control with respect to the image signal stored in the storage unit.

**12.** A medical image processing device comprising:

a selector unit for selectively outputting an image signal output from a medical control device for controlling a medical device equipped with an image pickup unit that outputs a captured subject image as the image signal; and a control unit for determining with respect to similarity between images among a plurality of images contained in an image group in accordance with the image pickup signal of two or more frames among image signals output from the medical control device based on a predetermined threshold value indicating a fluctuation between the images contained in the image group, and executes a predetermined control to allow the selector unit to output the image signal based on a result of the determination.

**13.** The medical image processing device according to Claim 12, wherein the control unit determines with respect to the similarity between adjacent images of the images contained in the image group based on the predetermined threshold value, and controls to extract the image signal of one frame from the image signals of the two or more consecutive frames based on the result of the determination so as to allow the selector unit to output the extracted image signal.

**14.** The medical image processing device according to Claim 12, wherein
the control unit determines with respect to the similarity between adjacent images of the images contained in the image group based on the predetermined threshold value; and
when a level of the similarity between one image of the adjacent images and the other consecutive image at a subsequent time point is low, the control unit controls to extract the one image, and to insert a predetermined number of sheets of the one image between the one image and the subsequent image to be output from the selector unit.

**15.** The medical image processing device according to any one of Claims 12 to 14, wherein the predetermined threshold value corresponds with an average value of a displacement value that indicates a displacement amount of a portion of the image at a previous time point in a portion of the other image at a subsequent time point among the images contained in the image group.

**16.** The medical image processing device according to any one of Claims 12 to 15, further comprising a storage unit for storing the image signal output from the medical control device, wherein the control unit executes the predetermined control with respect to the image signal stored in the storage unit.

**17.** A program allowing a computer that processes an image signal captured by a medical device equipped with an image pickup unit to execute:

a similarity detection procedure for determining with respect to the similarity between adjacent images of an image group including a plurality of images based on the image signals of two or more frames among those obtained by the medical device based on a predetermined threshold value that indicates the fluctuation between the images of the image group; and
an image signal extraction procedure extracting and outputting the image signal of one frame from those of two or more consecutive frames.

**18.** The program according to Claim 17, wherein the predetermined threshold value corresponds with an average value of a displacement value that indicates a displacement amount of a portion of the image at a previous time point in a portion of the other image at a subsequent time point among the images contained in the image group.

# FIG.1

# FIG.2

# FIG.3

# FIG.4

EP 1 977 676 A1

# FIG.5

START

S1 — n = 1

S14 — n = n+1

S2 — STORE IMAGE In IN STORAGE CIRCUIT 53

S3 — n = 1?

S3-1 — STORE IMAGE I1 IN STORAGE CIRCUIT 53a  ← YES

NO

S4 — i = 1, V = 0

S5 — VIRTUALLY DIVIDE IMAGE In-1 INTO N(N=2, 3, 4···) AREAS

S6 — DETECT POSITION WHERE AREA Hi ON IMAGE In-1 BECOMES MOST ADAPTABLE ON IMAGE In BASED ON LEVEL OF SIMILARITY

S9-1 — i = i+1

S7 — CALCULATE MOTION VECTOR Vi IN AREA Hi

S8 — V = V+|Vi|

S9 — i ≤ N?

YES

NO

S10 — Va = V/N

S11 — STORE IMAGE In IN STORAGE CIRCUIT 53a

S12 — Va < Vthr1?

S12-1 — DELETE IMAGE In FROM STORAGE CIRCUIT 53  ← YES

NO

S13 — IMAGE TO BE OUTPUT EXISTS?

YES

NO

END

23

# FIG.6

START

*S101*

n = 1

*S102*

STORE IMAGE In IN
STORAGE CIRCUIT 53

*S115*

n = n+1

*S103-1*

STORE IMAGE I1
IN STORAGE
CIRCUIT 53a

YES

*S103*

n = 1?

NO

*S104*

i = 1, V = 0, j = 0

*S105*

VIRTUALLY DIVIDE
IMAGE In-1 INTO
N(N=2, 3, 4···) AREAS

*S111*

Va = V/j

*S110-1*

i = i+1

*S106*

DETECT POSITION WHERE
AREA Hi ON IMAGE In-1
BECOMES MOST ADAPTABLE
ON IMAGE In BASED ON
LEVEL OF SIMILARITY

*S112*

STORE IMAGE In IN
STORAGE CIRCUIT 53a

*S107*

CALCULATE MOTION
VECTOR Vi IN AREA Hi

*S113*

*S113-1* YES

Va < Vthr2?

NO

*S108*

YES

Rthr < Ri?

NO *S109*

V = V+|Vi|, j = j+1

DELETE IMAGE In
FROM STORAGE
CIRCUIT 53

*S114*

YES

IMAGE TO BE
OUTPUT EXISTS?

NO

END

*S110*

YES

i ≤ N?

NO

# FIG.7

START — S201

n = 1 — S201

S216
n = n+1

STORE IMAGE In IN STORAGE CIRCUIT 53 — S202

n = 1? — S203

YES

S203-1
STORE IMAGE I1 IN STORAGE CIRCUIT 53a

NO

i = 1, V = 0, j = 0 — S204

VIRTUALLY DIVIDE IMAGE In-1 INTO N(N=2, 3, 4···) AREAS — S205

S210-1
i = i+1

DETECT POSITION WHERE AREA Hi ON IMAGE In-1 BECOMES MOST ADAPTABLE ON IMAGE In BASED ON LEVEL OF SIMILARITY — S206

Va = V/j — S211

STORE IMAGE In IN STORAGE CIRCUIT 53a — S212

CALCULATE MOTION VECTOR Vi IN AREA Hi — S207

j < jthr? — S213

NO

YES

Rthr2 < Ri? — S208

YES

Va < Vthr3? — S214

S214-1
YES

DELETE IMAGE In FROM STORAGE CIRCUIT 53

NO

NO

V = V+|Vi| AND j = j+1 — S209

IMAGE TO BE OUTPUT EXISTS? — S215

YES

i ≦ N? — S210

YES

NO

NO

END

25

# FIG.8

# FIG.9

START

$S401$ — n = 1, Vall = 0

$S416$ — n = n+1

$S402$ — STORE IMAGE In IN STORAGE CIRCUIT 53

$S403$ — n = 1?

YES → $S403-1$ — STORE IMAGE I1 IN STORAGE CIRCUIT 53a

NO

$S404$ — i = 1, V = 0

$S405$ — VIRTUALLY DIVIDE IMAGE In-1 INTO N(N=2, 3, 4···) AREAS

$S409-1$ — i = i+1

$S406$ — DETECT POSITION WHERE AREA Hi ON IMAGE In-1 BECOMES THE MOST ADAPTABLE ON IMAGE In BASED ON SIMILARITY

$S407$ — CALCULATE MOTION VECTOR Vi IN AREA Hi

$S408$ — V = V+|Vi|

$S409$ — i ≦ N?

YES

NO

$S410$ — Va = V/N

$S411$ — Vall = Va+Vall

$S412$ — STORE IMAGE In IN STORAGE CIRCUIT 53a

$S413$ — Va < Vthr5?

NO

YES

$S414$ — Vall < Vthr6?

NO

YES

$S414-2$ — Vall = 0

$S414-1$ — DELETE IMAGE In FROM STORAGE CIRCUIT 53

$S415$ — IMAGE TO BE OUTPUT EXISTS?

YES

NO

END

EP 1 977 676 A1

# FIG.10

In-1

In

# FIG.11

28

# FIG.12

START

S501 n = 1, j = 1, Vall = 0

S518 n = n+1

S502 READ IMAGE In FROM STORAGE CIRCUIT 92

S503 n = 1?

S503-1 list[1] = 1

S503-2 j = j+1

S504 i = 1, V = 0

S505 VIRTUALLY DIVIDE IMAGE In-1 INTO N(N=2, 3, 4···) AREAS

S506 DETECT POSITION WHERE AREA Hi ON IMAGE In-1 BECOMES MOST ADAPTABLE ON IMAGE In BASED ON LEVEL OF SIMILARITY

S509-1 i = i+1

S507 CALCULATE MOTION VECTOR Vi IN AREA Hi

S508 V = V+|Vi|

S509 i ≦ N?

S510 Va = V/N

S511 Vall = Va+Vall

S512 Va < Vthr7?

S513 Vall < Vthr8?

S514 EXTRACT FRAME NO. Fm OF IMAGE IFm

S515 CORRELATE FRAME NO. Fm WITH ALL IMAGE LISTS FROM jTH IMAGE LIST, LIST[j] TO (n-1)TH IMAGE LIST, LIST[n-1] TO BE STORED

S516 Vall = 0, j = n

S517 IMAGE TO BE READ EXISTS?

END

29

EP 1 977 676 A1

# FIG.13

START

S601 — n = 1, j = 1, k = 0, Vall = 0

S622 — n = n+1

S602 — READ IMAGE In FROM STORAGE CIRCUIT 92

S603 — n = 1?

YES → S603-1 — list[1] = 1 → S603-2 — j = j+1

NO

S604 — i = 1, V = 0

S615 — EXTRACT FRAME NO. Fm OF IMAGE IFm

S616 — CORRELATE FRAME NO. Fm WITH ALL IMAGE LISTS FROM (j+k)TH IMAGE LIST, LIST[j+k] TO (n-1+k)TH IMAGE LIST, LIST[n-1+k] TO BE STORED

S617 — Vall = 0, j = n

S618 — OBTAIN NUMBER OF IMAGES I DISPLAYED BETWEEN IMAGES In-1 AND In BASED ON Va

S619 — CORRELATE FRAME NO. (n-1) WITH ALL IMAGE LISTS FROM (j+k)TH IMAGE LIST, LIST[j+k] TO (n-1+k)TH IMAGE LIST, LIST[n-1+k] TO BE STORED

S620 — k = k+I

S621 — IMAGE TO BE READ EXISTS?

YES → (loop to top)

NO → END

S605 — VIRTUALLY DIVIDE IMAGE In-1 INTO N(N=2, 3, 4···) AREAS

S609-1 — i = i+1

S606 — DETECT POSITION WHERE AREA Hi ON IMAGE In-1 BECOMES MOST ADAPTABLE ON IMAGE In BASED ON LEVEL OF SIMILARITY

S607 — CALCULATE MOTION VECTOR Vi IN AREA Hi

S608 — V = V+|Vi|

S609 — i ≤ N?

YES → (loop to S609-1)

NO

S610 — Va = V/N

S611 — Vall = Va+Vall

S612 — Va < Vthr7?

NO → (loop)

YES

S613 — Vall < Vthr8?

YES → (to S620 k = k+I)

NO → (loop)

30

# FIG.14

OCCURENCE

$\theta$

0    45    90    - - - - - - - - - -    360

# FIG.15

$I_1$ $I_2$ $I_3$ $I_4$ $I_5$ $I_6$ $I_7$    - - - - - - - - - -    $I_{n-4}$  $I_{n-2}$  $I_n$

$I_{n-5}$  $I_{n-3}$  $I_{n-1}$

TIME

# FIG.16

I$_1$ I$_2$ I$_3$ I$_4$ I$_5$ I$_6$ I$_7$

I$_{n-5}$ I$_{n-4}$ I$_{n-2}$ I$_n$
I$_{n-3}$ I$_{n-1}$

0                                                                                    DISTANCE

IMAGE GROUP WITH
SMALL FLUCTUATION

IMAGE GROUP WITH
SMALL FLUCTUATION

# FIG.17

I$_1$ I$_4$ I$_4$ I$_4$ I$_4$ I$_4$ I$_7$

I$_{n-3}$ I$_{n-3}$ I$_n$
I$_{n-5}$ I$_{n-3}$ I$_{n-3}$

TIME

# FIG.18

Va3

Va2

Va4

GRAVITY
CENTER

Va5

Va6

# FIG.19

$I_1$ $I_2$ $I_3$ $I_4$ $I_5$ $I_6$ $I_7$

------

$I_{n-4}$ $I_{n-2}$ $I_n$

$I_{n-5}$ $I_{n-3}$ $I_{n-1}$

TIME

# FIG.20

$I_1$  $I_2$ $I_3$ $I_4$  $I_5$ $I_6$ $I_7$

$I_{n-5}$

$I_{n-4}$  $I_{n-2}$  $I_n$
$I_{n-3}$ $I_{n-1}$

0

DISTANCE

IMAGE GROUP WITH
SMALL FLUCTUATION

GREAT FLUCTUATION
AMONG IMAGES

# FIG.21

$I_1$ $I_4$ $I_4$ $I_4$ $I_4$ $I_4$ $I_7$

$I_{n-5}$  $I_{n-5}$  $I_{n-3}$  $I_{n-3}$  $I_n$
$I_{n-5}$  $I_{n-5}$  $I_{n-5}$  $I_{n-3}$  $I_{n-3}$

TIME

## INTERNATIONAL SEARCH REPORT

| International application No. |
| --- |
| PCT/JP2006/322815 |

A. CLASSIFICATION OF SUBJECT MATTER
*A61B1/00*(2006.01)i, *G06T1/00*(2006.01)i, *G06T7/20*(2006.01)i, *A61B5/07*
(2006.01)n

According to International Patent Classification (IPC) or to both national classification and IPC

B. FIELDS SEARCHED

Minimum documentation searched (classification system followed by classification symbols)
A61B1/00, G06T1/00, G06T7/20, A61B5/07

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2004-521662 A (Given Imaging Ltd.), 22 July, 2004 (22.07.04), | 1,2,4,5,8,9, 11 |
| Y | Claims 8 to 16, 23 to 28; Par. Nos. [0039] to [0057] & US 6709387 B & EP 1401512 A & WO 2001/087377 A2 | 3,6,7,10, 12-18 |
| Y | JP 8-251540 A (Matsushita Electric Industrial Co., Ltd.), 27 September, 1996 (27.09.96), Par. Nos. [0071] to [0076] (Family: none) | 3,6,7,10, 12-18 |

☒ Further documents are listed in the continuation of Box C. ☐ See patent family annex.

| * | Special categories of cited documents: |
| --- | --- |
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 30 November, 2006 (30.11.06) | 12 December, 2006 (12.12.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/322815

C (Continuation).   DOCUMENTS CONSIDERED TO BE RELEVANT

| Category* | Citation of document, with indication, where appropriate, of the relevant passages | Relevant to claim No. |
|---|---|---|
| A | US 3723644 A  (Bell Telephone Laboratories, Inc.),<br>27 March, 1973 (27.03.73),<br>Full text<br>(Family: none) | 1-18 |
| A | JP 2004-215155 A  (Ricoh Co., Ltd.),<br>29 July, 2004 (29.07.04),<br>Full text<br>(Family: none) | 1-18 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004000645 A
- JP 2006014444 A